(19) Europäisches Patentamt
European Patent Office
Office européen des brevets

(11) EP 4 707 366 A1

(12) **EUROPEAN PATENT APPLICATION**

(43) Date of publication:
**11.03.2026 Bulletin 2026/11**

(21) Application number: **24223146.2**

(22) Date of filing: **24.12.2024**

(51) International Patent Classification (IPC):
***C11D 1/37*** *(2006.01)* ***C07C 303/24*** *(2006.01)*
*C11D 1/29* *(2006.01)* *C11D 1/14* *(2006.01)*

(52) Cooperative Patent Classification (CPC):
(C-Sets available)
**C11D 1/37; C07C 303/24;** C11D 1/146; C11D 1/29
(Cont.)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **06.09.2024 EP 24198899**
**06.09.2024 EP 24198909**

(71) Applicant: **The Procter & Gamble Company**
**Cincinnati, OH 45202 (US)**

(72) Inventors:
- **BILLIAUW, Jan Julien Marie-Louise**
  **1853 Strombeek-Bever (BE)**
- **KEULEERS, Robby Renilde Francois**
  **1853 Strombeek-Bever (BE)**
- **VINSON, Phillip Kyle**
  **Cincinnati, 45202 (US)**

(74) Representative: **P&G Patent Belgium UK**
**N.V. Procter & Gamble Services Company S.A.**
**Temselaan 100**
**1853 Strombeek-Bever (BE)**

(54) **PROCESS FOR MAKING CONCENTRATED SURFACTANT BLENDS**

(57) The need for processes for forming flowable concentrated anionic surfactant blends having reduced levels of dioxane by-product while avoiding malodour and discoloration, and providing good low temperature stability, and useful for creating detergent composition with high performance and sudsing, without requiring high levels of solvent, is met by first forming an alcohol stream comprising at least one alkyl glycol and at least one alkyl alcohol, and then sulfating and neutralizing the alcohol stream to form the concentrated anionic surfactant blend.

EP 4 707 366 A1

(52) Cooperative Patent Classification (CPC): (Cont.)

C-Sets
**C07C 303/24, C07C 305/06;**
**C07C 303/24, C07C 305/10**

## Description

FIELD OF THE INVENTION

The present invention relates to processes for making concentrated surfactant blends, in particular, concentrated surfactant blends for use in making liquid detergent compositions, especially liquid hand dishwashing cleaning compositions.

BACKGROUND OF THE INVENTION

Liquid hand dishwashing detergents should be high sudsing and provide long-lasting suds. As such, liquid hand dishwashing compositions are typically formulated using alkyl alkoxylated sulfate anionic surfactants. Alkyl alkoxylated sulfate anionic surfactants are known for their effectiveness in generating substantial suds while efficiently removing grease, as well as providing good low-temperature stability.

The synthesis of alkyl alkoxylated sulfate anionic surfactants generally involves a multi-step process: an initial alkoxylation phase whereby alkyl alcohol undergoes alkoxylation, followed by a sulfation phase, and concluding with a neutralization phase. This sequence is crucial as alkyl alkoxylated sulfuric acids exhibit poor hydrolytic stability. Upon formation, these surfactants can be blended with non-alkoxylated alkyl sulfated anionic surfactants to achieve the desired average alkoxylation levels. Additionally, various alkyl alkoxylated sulfate and optionally unalkoxylated alkyl sulfate anionic surfactants may be combined to tailor the average alkyl chain length, degree of branching, or branching distribution. In addition, the synthesis process is challenging to control. In particular, the viscosity of the alkyl alcohol blend can significantly affect the subsequent sulfation step, with a higher viscosity requiring higher temperatures or reduced throughput during sulfation, while also making the colour and odour of the subsequent concentrated anionic surfactant blend harder to control. Higher temperature also results in higher residual amounts of 1,4-dioxane.

Making of the hand dishwashing composition can also be eased if the concentrated anionic surfactant blend has a lower viscosity. Typically, this is achieved through the addition of solvent such as ethanol. However, the addition of such solvents increases processing complexity and cost due to the increased flammability, while typically also having a detrimental effect on the composition viscosity. Achieving the desired concentrated surfactant blend viscosity is particularly challenging for blends comprising lowly branched anionic surfactant. It is also desirable to limit the need to add processing ingredients such as solvents in order to improve the environmental sustainability profile of the surfactant blend and compositions made using the blend. Furthermore, by limiting or eliminating solvent from the surfactant blend, less salt needs to be added to the composition made using the blend, to achieve the desired composition viscosity, while also limiting the need for corrosion-resistant materials for the making process. A lower viscosity of the concentrated anionic surfactant blend also allows faster production of the resultant liquid hand dishwashing composition, especially where the composition is made via continuous liquid processing methods.

When making concentrated anionic surfactant blends comprising alkoxylated, and especially ethoxylated alkyl sulfate surfactants, 1,4-dioxanes can be produced. Tight control of processing conditions and feedstock material compositions is typically needed, both during alkoxylation especially ethoxylation and sulfation steps, so that the amount of 1,4-dioxane by-product within alkoxylated especially ethoxylated alkyl sulfates can be minimised. Even if the 1,4-dioxane by-product level is kept to a minimum in the freshly formed surfactant blend, for many blends formed from prior art processes, the level of 1,4-dioxane by-product increases over time. The formation of 1,4-dioxanes has been found to be higher when concentrated alkyl ethoxylated sulfate blends are stored at higher pH.

Moreover, while non-alkoxylated surfactants offer an alternative to alkyl alkoxylated sulfate anionic surfactants, they do not provide the same sudsing and low temperature stability benefits.

Consequently, there remains a need for concentrated anionic surfactant blends and processes for making them, that provide similar performance to traditional anionic surfactant blends comprising alkyl alkoxylated sulfate anionic surfactant, while being readily flowable without requiring significant solvent levels. There is a further need that such concentrated anionic surfactant blends are made through a process utilising an alcohol stream that is easier to sulfate, while resulting in a blend which is less prone to discoloration or malodour and easier to process. There is also a need for the process, and resultant concentrated anionic surfactant blend to comprise reduced levels or even substantial absence of 1,4-dioxane by-product.

WO2024063990A1 relates to an aqueous light duty liquid detergent formulation including: water; a zwitterionic surfactant; and an alcohol ethoxysulfate surfactant in which 95 to 100 mol% of the alcohol ethoxysulfate surfactant has a degree of ethoxylation of 1. WO202463991A1 relates to an aqueous laundry detergent composition is provided including: water; a cleaning surfactant; wherein the cleaning surfactant comprises a blend of a nonionic surfactant and an anionic surfactant; wherein the anionic surfactant comprises an alcohol ethoxysulfate surfactant in which 95 to 100 mol% of the alcohol ethoxysulfate surfactant has a degree of ethoxylation of 1. While referred to as alcohol "ethoxysulfate" surfactants, the surfactants described in WO2024063990A1 and WO202463991A1 are not derived through processes which include

an ethoxylation step, instead reacting an ethylene glycol monomer or oligomer with an alkene, before sulfation. Exemplary processes for making such "alkyl glycol" sulfates are described in WO202464645A. In contrast to alkyl ethoxylated sulfate anionic surfactants, alkyl glycol sulfate anionic surfactants are typically formed via the acid-catalysed addition of the glycol which results in the Markovnikov addition of a hydrogen (typically on the less substituted side) and an alkoxy group, resulting in the addition of an ether-bond, followed by a reaction to add SO3, i.e., a sulfation process, and subsequent neutralization to provide a counterion. This results in the alkyl glycol sulfate anionic surfactants comprising an extremely narrow distribution of ethylene glycol units in the alkyl glycol sulfate d anionic surfactant, typically from 1 to 3 ethylene glycol units per molecule of alkyl glycol sulfate anionic surfactant, or even 1 ethylene glycol unit per molecule of alkyl glycol sulfate anionic surfactant. The alkyl glycol sulfate anionic surfactant formed using such processes is also typically free of alkyl sulfate anionic surfactant that does not comprise the glycol linking group between the alkyl chain and the sulfate group. WO202464645A1 describes a process which includes the steps of contacting an olefin, an alcohol and a metallosilicate catalyst to form oligomers of an alcohol "ethoxylate" in which 95 to 100 mol% of the alcohol ethoxylate has a degree of ethoxylation of 1, and then sulphating the oligomers.

JP200610443 8A provides a liquid detergent composition providing detergency against oily stains, rinsability, handleability and handling safety, the liquid detergent composition comprises an anionic surfactant, a nonionic surfactant and water, in which the anionic surfactant comprises a secondary alkyl ether sulfate salt. JP2006137872A provides a powdery detergent composition having good fluidity, detergency and rinsing property, having little odor and excellent in disperse solubility even to low-temperature water, the powdery detergent composition comprises a higher secondary alcohol alkoxylate sulfate salt and an anionic surfactant other than the higher secondary alcohol alkoxylate sulfate salt. EP0850907A1 relates to a higher secondary alcohol alkoxylate compound composition, a method for the production thereof, and a detergent and an emulsifier using the composition.

The following journal articles describe processes whereby alkoxylate surfactants are derived from alpha-olefins: BAKKER P M: "Sulfonates and sulfates of sec-alkyl ethyl ether: detergents prepared by the addition of substituted alcohols to 1-alkenes", CHIMIE, PHYSIQUE ET APPLICATIONS PRATIQUES DES AGENTS DE SURFACT, XX, XX, 9 September 1968 (1968-09-09), pages 157 - 165, XP002075332; J. F. KNIFTON: "Detergent-range alcohol alkoxylates via vicinal glycol additions to alpha-olefins" APPLIED CATALYSIS, A, vol. 130, 1995, pages 79-88, XP002075333; P. M. BAKKER: "An exploratory study of the addition reactions of ethyleneglycol, 2-chloroethanol and 1,3-dichloro-2-propanol to 1-dodecene" JOURNAL OF THE AMERICAN OIL CHEMISTS' SOCIETY, vol. 44, September 1967, pages 517-521, XP002058548.

## SUMMARY OF THE INVENTION

The present invention relates to a process for making a concentrated surfactant blend, wherein the concentrated surfactant blend comprises a blend of at least one alkyl sulfated anionic surfactant and at least one alkyl glycol sulfate anionic surfactant, wherein the process comprises the following steps: providing an alcohol stream, wherein the alcohol stream comprises: at least one alkyl glycol and at least one alkyl alcohol, wherein: the alkyl glycol has the formula: R1CH(R2)(OCH2CH2)nOH, wherein: R1 is independently H, alkyl, alkylene, or a mixture thereof; R2 is independently alkyl, alkylene, or a mixture thereof; the sum of the carbon atoms present in R1 and R2 is on average from 7 to 19; n is from 1 to 3, wherein 90 mol% or greater of the alkyl glycol of structure (I) have an n of 1 and 10 mol% or less of the alkyl glycol of structure (I) have an n of 2 or greater; sulphating the alcohol stream to provide an alkyl sulfuric acid stream comprising at least one alkyl sulphuric acid and at least one alkyl glycol sulphuric acid; neutralising the alkyl sulfuric acid stream to provide a concentrated surfactant blend comprising the at least one alkyl sulfated anionic surfactant and the at least one alkyl glycol sulfate anionic surfactant, wherein: the resultant concentrated surfactant blend has a pH of at least 7.0 when measured as a 10% by weight solution of the concentrated surfactant blend in demineralized water at 20 °C.

The present invention further relates to a concentrated surfactant blend, wherein the concentrated surfactant blend comprises anionic surfactant at a level of from 20% to 80%, wherein the concentrated surfactant blend comprises: alkyl glycol sulfate anionic surfactant, wherein the alkyl glycol sulfate anionic surfactant has the formula: R1CH(R2)(OCH2CH2)nOSO3-$M^+$, wherein: R1 is independently H, alkyl, alkylene, or a mixture thereof; R2 is independently alkyl, alkylene, or a mixture thereof; the sum of the carbon atoms present in R1 and R2 is on average from 7 to 19; n is from 1 to 3, wherein 90 mol% or greater of the surfactant molecules of structure (I) have an n of 1 and 10 mol% or less of the surfactant molecules of structure (I) have an n of 2 or greater; and M+ is a counterion; and alkyl sulfated anionic surfactant, wherein the alkyl sulfated anionic surfactant has a degree of alkoxylation of less than 3.0.

## DETAILED DESCRIPTION OF THE INVENTION

The present invention relates to a novel process for producing a concentrated anionic surfactant blend characterized by a significantly reduced fraction of alkyl alkoxylated sulfate anionic surfactant isomers having a degree of alkoxylation of 2 or greater. By reducing the amount of alkyl alkoxylated sulfate anionic surfactant isomers having a

degree of alkoxylation of 2 or greater, the desired average degree of ethoxylation is maintained while markedly decreasing the formation of 1,4-dioxane by-products.

Moreover, the use of this specific alcohol stream in the present process is believed to contribute to a lower viscosity, which facilitates improvements in the subsequent sulfation step. This is particularly advantageous when employing a liquid-gas interface reactor, such as a falling film reactor, as it allows for lower processing temperatures and increased throughput during sulfation in the falling film reactor, while avoiding discoloration and malodour, while also reducing the formation of 1,4-dioxane.

Additionally, the resulting liquid hand dishwashing compositions can exhibit a reduced Krafft point, which enhances surfactant solubility at lower temperatures. This reduction in the Krafft point also translates to a decreased physical stability sensitivity for added salts, thereby achieving the desired low-temperature stability and performance with minimal additional solvent content. In particular, a higher Kraft point can mean faster separation of the surfactant into a high active phase at higher salt levels, necessitating the need for additional solvent. Consequently, the concentrated anionic surfactant blend allows for the formulation of liquid hand dishwashing detergents that necessitate less solvent, such as ethanol, to achieve the target viscosity, resulting in more efficient and effective detergent formulations, without the disadvantages in presence of high salt levels.

As used herein, articles such as "a" and "an" when used in a claim, are understood to mean one or more of what is claimed or described.

The term "comprising" as used herein means that steps and ingredients other than those specifically mentioned can be added. This term encompasses the terms "consisting of" and "consisting essentially of." The compositions of the present invention can comprise, consist of, and consist essentially of the essential elements and limitations of the invention described herein, as well as any of the additional or optional ingredients, components, steps, or limitations described herein.

The term "dishware" as used herein includes cookware and tableware made from, by non-limiting examples, ceramic, china, metal, glass, plastic (e.g., polyethylene, polypropylene, polystyrene, etc.) and wood.

The term "grease" or "greasy" as used herein means materials comprising at least in part (*i.e.*, at least 0.5 wt% by weight of the grease in the material) saturated and unsaturated fats and oils, preferably oils and fats derived from animal sources such as beef, pig and/or chicken.

The terms "include", "includes" and "including" are meant to be non-limiting.

The term "particulate soils" as used herein means inorganic and especially organic, solid soil particles, especially food particles, such as for non-limiting examples: finely divided elemental carbon, baked grease particle, and meat particles.

The term "sudsing profile" as used herein refers to the properties of a cleaning composition relating to suds character during the dishwashing process. The term "sudsing profile" of a cleaning composition includes initial suds volume generated upon dissolving and agitation, typically manual agitation, of the cleaning composition in the aqueous washing solution, and the retention of the suds during the dishwashing process. Preferably, hand dishwashing cleaning compositions characterized as having "good sudsing profile" tend to have high initial suds volume and/or sustained suds volume, particularly during a substantial portion of or for the entire manual dishwashing process. This is important as the consumer uses high suds as an indicator that enough cleaning composition has been dosed. Moreover, the consumer also uses the sustained suds volume as an indicator that enough active cleaning ingredients (e.g., surfactants) are present, even towards the end of the dishwashing process. The consumer usually renews the washing solution when the sudsing subsides. Thus, a low sudsing cleaning composition will tend to be replaced by the consumer more frequently than is necessary because of the low sudsing level.

It is understood that the test methods that are disclosed in the Test Methods Section of the present application must be used to determine the respective values of the parameters of Applicants' inventions as described and claimed herein.

All percentages are by weight of the total composition, as evident by the context, unless specifically stated otherwise. All ratios are weight ratios, unless specifically stated otherwise, and all measurements are made at 25°C, unless otherwise designated.

Process for making a concentrated surfactant blend

The present process is used to make a concentrated surfactant blend. The concentrated surfactant blend comprises a blend of at least one alkyl glycol sulfate anionic surfactant and at least one alkyl sulfated anionic surfactant. The alkyl sulfated anionic surfactant can be at least partially alkoxylated, but is preferably free of alkoxylation. The process comprises at least the following steps: providing an alcohol stream comprising a blend of at least one alkyl glycol and at least one alkyl alcohol; sulphating the alcohol stream to provide an alkyl sulfuric acid stream comprising at least one alkyl glycol sulphuric acid and at least one alkyl sulphuric acid; and neutralising the alkyl sulfuric acid stream to provide a concentrated anionic surfactant blend comprising the at least one alkyl glycol sulfate anionic surfactant and the at least one

alkyl sulfated anionic surfactant.

The concentrated surfactant blends produced by the processes described herein are typically more readily flowable. As such, little or no non-aqueous solvent is typically added to the concentrated surfactant blend. As such, the concentrated surfactant blend comprises less than 15%, preferably less than 10%, more preferably less than 5% by weight or may even be essentially free or free of non-aqueous solvent. If added, the non-aqueous solvent is typically added during or after the neutralisation step.

Providing the alcohol stream

The alcohol stream is provided by blending together an alkyl glycol with an alkyl alcohol, both as described herein. The alcohol in the alcohol stream comprises, and can consist of the alkyl glycol and the alkyl alcohol.

In the alcohol stream, the alkyl glycol and the alkyl alcohol can be present in a weight ratio of from 1:8 to 2:1, preferably from 1:7 to 1:1, more preferably from 1:5 to 1:2.

The formation of sulfuric acid can be minimised and a low ionic strength maintained, when the alkyl alcohol stream fed into the sulfation reaction preferably comprises less than 0.1%, preferably less than 0.05%, more preferably is free of water.

Alkyl glycol

At least one alkyl glycol and at least one alkyl alcohol are blended together to provide the alcohol stream.

The alcohol stream comprises an alkyl glycol having the formula:

$$R1CH(R2)(OCH_2CH_2)_nOH \qquad (I)$$

wherein:

R1 is independently H, alkyl, alkylene, or a mixture thereof;
R2 is independently alkyl, alkylene, or a mixture thereof;
the sum of the carbon atoms present in R1 and R2 is on average from 7 to 19;
n is from 1 to 3, wherein 90 mol% or greater of the alkyl glycol of structure (I) have an n of 1 and 10 mol% or less of the alkyl glycol of structure (I) have an n of 2 or greater.

The value of n in structure (I) has a value from 1 to 3. For example, n may be 1, 2, or 3. 90 mol% or greater of the alkyl glycol of structure (I) have an n of 1 and 10 mol% or less of the alkyl glycol of structure (I) have an n of 2 or greater. More preferably, 92 mol% or greater of the alkyl glycol of structure (I) have an n of 1 and 8 mol% or less of the alkyl glycol of structure (I) have an n of 2 or greater. Most preferably, 95 mol% or greater of the alkyl glycol of structure (I) have an n of 1 and 5 mol% or less of the alkyl glycol of structure (I) have an n of 2 or greater.

In formula (I), the group $(OCH_2CH_2)_n$ can be derived from ethylene glycol (ethane-1,2-diol) or oligomers of ethylene glycol, wherein the suitable oligomers of ethylene glycol comprise up to 3 monomer units of the ethylene glycol. Preferably in formula (I), the group $(OCH_2CH_2)_n$ is derived from ethylene glycol.

R1 is independently H, alkyl, alkylene, or a mixture thereof, preferably H, alkyl, or a mixture thereof, with a blend of H and alkyl being particularly preferred. Typically, the alkyl glycol used to make the alkyl glycol sulfate anionic surfactant is a blend of alkyl glycol of formula (I), in which R1 is H and alkyl, with the major part being alkyl glycol of formula (I), in which R1 is alkyl, with a relatively minor part being alkyl glycol of formula (I), in which R1 is H. This is because the alkyl glycol of formula (I) are typically formed via an addition reaction which follows Markovnikov's rule, resulting in the most stable carbocation being formed on the more substituted carbon atom of the alkene bond due to induction and hyperconjugation. However, residual amounts of alkyl glycol of formula (I) are formed in which R1 is H, since other less substituted, less stable carbocation are still formed as intermediates at some residual concentration. Preferably in at least 50% by weight, more preferably at least 60%, most preferably at least 80% by weight of the alkyl glycol of formula (I), R1 is alkyl, with the remainder being H. R1 preferably comprises from 1 to 6 carbon atoms, preferably from 1 to 3 carbon atoms. Preferably, in at least 80%, more preferably in at least 90% by mol of the alkyl glycol of formula (I), R1 comprises 1 carbon atom, more preferably is methyl. As such, branching, and especially methyl-branching, at the C1 position is preferably present for at least 80%, or even at least 90% by mol of alkyl glycol sulfate anionic surfactant, wherein the C1 position is the carbon atom bound to the oxygen atom of the glycol sulfate group.

R2 is independently alkyl, alkylene, or a mixture thereof, preferably alkyl. R2 preferably comprises on average from 6 to 19 carbon atoms, preferably from 8 to 17 carbon atoms, more preferably from 9 to 15 carbon atoms, most preferably 10 to 13 carbon atoms. R2 can be linear, branched or a combination thereof, with R2 being preferably linear.

The sum of the carbon atoms present in R1 and R2 is on average from 7 to 19, preferably from 9 to 17, more

preferably from 11 to 15, most preferably 11 to 13.

For both R1 and R2, alkyl is preferred over alkylene as the presence of multiple double-bonds in the starting alkene can lead to reduced selectivity for the terminal double bond during the reaction to form the ether-bond. As such, while R1 and R2 can be independently saturated or unsaturated, saturated is preferred.

The alkyl glycol can have a mol average alkyl chain length of from 8 to 18, preferably from 10 to 14, most preferably from 12 to 13 carbon atoms, as defined by R1CH(R2).

The distribution of R1 and R2 can be determined either analytically (for instance through $^{1}$H NMR and/or $^{13}$C NMR) or can be determined from the starting materials used to make the alkyl glycol sulfate anionic surfactant.

The alkyl glycol can be derived from at least one alkene, preferably at least one linear alpha olefin (LAO), more preferably wherein the linear alpha olefin is selected from the group consisting of: 1-dodecene, 1-tetradecene, 1-hexadecene, and mixtures thereof. In formula (I), R1CH(R2) can be derived from suitable alkenes, especially linear alpha olefin (LAO). Linear alpha olefins are straight-chain terminal alkenes and hence comprise a double bond at the alpha ($\alpha$-, 1- or primary) position, and a linear (unbranched) hydrocarbon chain. As such, they result in longer, less branched, alkyl groups in the resultant alkyl glycol sulfate anionic surfactant relative to when starting from mid-chain unsaturated alkenes. Suitable linear alpha olefins can be selected from the group consisting of: 1-dodecene, 1-tetradecene, 1-hexadecene, and mixtures thereof. Commercial sources of such alpha olefins can contain minor levels of other chain-length olefins, branched olefins, and vinylidene olefins.

Linear alpha olefins can be made using any known process, including via: the oligomerization of ethylene, Fischer-Tropsch synthesis, dehydration of alcohols, or thermal cracking of waxes. Alternatively, the olefin can be "bio-derived", as described in WO2011002284A or Yu, H., Wang, C., Lin, T. et al. "Direct production of olefins from syngas with ultrahigh carbon efficiency", Nat. Commun. 13, 5987 (2022). Alkyl glycol sulfate anionic surfactants are similar to alkyl ethoxylated sulfate anionic surfactants but have some key differences.

Alternatively, branched olefins can be used. The chemical and physical properties of the resultant alkyl glycol sulfate anionic surfactant can vary with the choice of linear or branched olefin, and also with the degree and type of branching. Examples of suitable branched olefins include propylene tetramer and butylene trimer (branched dodecene), as used in the manufacturing of oxo-alcohols by Exxon (Exxal 13™) and BASF (Lutensol TDA®).

In contrast to alkyl ethoxylated alcohols used to make alkyl ethoxylated sulfate anionic surfactants, alkyl glycols used to make alkyl glycol sulfate anionic surfactants are typically formed via the acid-catalysed addition of the glycol which results in the Markovnikov addition of a hydrogen (typically on the less substituted side) and an alkoxy group, resulting in the addition of an ether-bond.

This results in the alkyl glycol and subsequent alkyl glycol sulfate anionic surfactants comprising an extremely narrow distribution of ethylene glycol units in the alkyl glycol sulfate anionic surfactant, typically from 1 to 3 ethylene glycol units per molecule of alkyl glycol sulfate anionic surfactant, or even 1 ethylene glycol unit per molecule of alkyl glycol sulfate anionic surfactant. The alkyl glycol formed using such processes is also typically free or substantially free of alkyl alcohols that do not comprise the glycol linking group between the alkyl chain and the hydroxyl group.

When the alkyl glycol is derived from the reaction of an alpha-olefin with the ethylene glycol, methyl branching at the C1 position can be present for at least 90%, or even at least 95% by mol of the alkyl glycol and subsequent alkyl glycol sulfate anionic surfactant, wherein the C1 position is the carbon atom bound to the oxygen atom of the glycol group.

Alkyl alcohol

The mol average alkyl chain length of the alkyl alcohol can be from 8 to 18, preferably from 10 to 14, more preferably from 12 to 14, most preferably from 12 to 13 carbon atoms, to provide a combination of improved grease removal and enhanced speed of cleaning.

The alkyl chain of the alkyl alcohol can have a mol fraction of C12 and C13 chains of at least 50%, preferably at least 65%, more preferably at least 80%, most preferably at least 90%. Suds mileage is particularly improved, especially in the presence of greasy soils, when the C13/C12 mol ratio of the alkyl chain is at least 57/43, preferably from 60/40 to 90/10, more preferably from 60/40 to 80/20, most preferably from 60/40 to 70/30, while not compromising suds mileage in the presence of particulate soils.

The relative molar amounts of C 13 and C12 alkyl chains in the alkyl alcohol can be derived from the carbon chain length distribution of the surfactants. The carbon chain length distributions of the alkyl chains of the alkyl alcohol can be obtained from the technical data sheets of the constituent alkyl alcohols. Alternatively, the chain length distribution and average molecular weight of the alkyl alcohol can also be determined by methods known in the art. Such methods include capillary gas chromatography with flame ionization detection on medium polar capillary column, using hexane as the solvent. The chain length distribution is based on the starting alcohol and/or alkoxylated alcohol. As such, the alkyl sulfate anionic surfactant should be hydrolyzed back to the corresponding alkyl alcohol and alkyl alkoxylated alcohol before analysis, for instance using hydrochloric acid.

The alkyl alcohol can be alkoxylated or free of alkoxylation. When alkoxylated, the alkyl alcohol can have an

average degree of alkoxylation of less than 1.0, preferably less than 0.5, more preferably less than 0.25, even more preferably less than 0.1, with no alkoxylation being particularly preferred. When alkoxylated, ethoxylation is preferred. As such, the alkyl alcohol can comprise less than 10% preferably less than 5% by weight of the alkyl alcohol of an alkoxylated alcohol, more preferably wherein the alkyl alcohol is free of an alkoxylated alcohol.

The average degree of alkoxylation is the mol average degree of alkoxylation (i.e., mol average alkoxylation degree) of all the alkyl sulfate anionic surfactant. Hence, when calculating the mol average alkoxylation degree of the alkyl sulfate anionic surfactant, the mols of non-alkoxylated sulfate anionic surfactant are included. The mols of alky glycol sulfate anionic surfactant are excluded.

Mol average alkoxylation degree = (x1 * alkoxylation degree of surfactant 1 + x2 * alkoxylation degree of surfactant 2 + ....) / (x1 + x2 + ....)

where x1, x2, ... are the number of moles of each alkyl (or alkoxy) sulfate anionic surfactant of the mixture and alkoxylation degree is the number of alkoxy groups in each alkyl sulfate anionic surfactant.

Preferred alkyl alkoxy sulfates are alkyl ethoxy sulfates.

Ethoxylated alkyl sulfate anionic surfactants are typically formed by first ethoxylating an alkyl alcohol to add ethylene oxide ($C_2H_4O$) to the alkyl alcohol. The resultant alkyl ethoxylated nonionic surfactant is then sulfated to form the alkyl ethoxylated sulfate anionic surfactant.

When forming the alkyl ethoxylated nonionic surfactant, some polymerisation of the ethylene oxides occurs which results in a relatively broad distribution of degree of ethoxylation of the alkyl alcohol. The ethylene oxide used to make ethoxylated surfactants, can also give rise to the formation of 1,4-dioxanes, especially where the molecules of the alkyl ethoxylated sulfate anionic surfactant comprise at least two EO groups. This is because the ethylene oxide must first dimerise to form 1,4-dioxane. With alkyl ethoxylated sulfate anionic surfactants, without wishing to be bound by theory, through tight control of processing conditions and feedstock material compositions, both during alkoxylation especially ethoxylation and sulfation steps, the amount of 1,4-dioxane by-product within alkoxylated especially ethoxylated alkyl sulfates can be reduced. Based on recent advances in technology, a further reduction of 1,4-dioxane by-product can be achieved by subsequent stripping, distillation, evaporation, centrifugation, microwave irradiation, molecular sieving or catalytic or enzymatic degradation steps. Processes to control 1,4-dioxane content within alkoxylated/ethoxylated alkyl sulfates have been described extensively in the art. Alternatively 1,4-dioxane level control within detergent formulations has also been described in the art through addition of 1,4-dioxane inhibitors to 1,4-dioxane comprising formulations, such as 5,6-dihydro-3-(4-morpholinyl)-1-[4-(2-oxo-1-piperidinyl)-phenyl]-2-(1-H)-pyridone, 3-$\alpha$-hydroxy-7-oxo stereoisomer-mixtures of cholinic acid, 3-(N- methyl amino)-L-alanine, and mixtures thereof. However, it remains challenging to further reduce the dioxane level in alkyl ethoxylated surfactants.

The performance can be affected by the width of the alkoxylation distribution of the alkoxylated alkyl sulfate anionic surfactant, including grease cleaning, sudsing, low temperature stability and viscosity of the finished product. The alkoxylation distribution, including its broadness can be varied through the selection of catalyst and process conditions when making the alkoxylated alkyl alcohol.

Non-alkoxylated alkyl sulfate surfactants can be formed using naturally derived alkyl chains, such as those derived from palm oil or coconut oil. It has also been found that such non-alkoxylated alkyl sulfate surfactants are more readily biodegradable by microorganisms in soil and natural waters.

The alkyl alcohol preferably has an average degree of branching of less than 60%, preferably less than 40%, preferably less than 20%, most preferably less than 10%. Particularly preferred, the alkyl alcohol is linear. Linear alkyl chains are typically derived from renewable sources.

The alkyl sulfate anionic surfactant and the alkyl alkoxy sulfate anionic surfactant can comprise at least 5%, preferably at least 10%, most preferably at least 25%, by weight of the surfactant, of branching on the C2 position (as measured counting carbon atoms from the sulfate group for non-alkoxylated alkyl sulfate anionic surfactants and counting from the alkoxy-group furthest from the sulfate group for alkoxylated alkyl sulfate anionic surfactants). More preferably, greater than 75%, even more preferably greater than 90%, by weight of the total branched alkyl content consists of C1-C5 alkyl moiety, preferably C1-C2 alkyl moiety. It has been found that formulating the inventive compositions using alkyl sulfate surfactants or alkyl alkoxy sulfate surfactants having the aforementioned degree of branching results in improved low temperature stability. Such compositions require less solvent in order to achieve good physical stability at low temperatures. As such, the compositions can comprise lower levels of organic solvent, such as less than 5.0% by weight of the liquid composition of organic solvent, while still having improved low temperature stability. Higher surfactant branching also provides faster initial suds generation, but typically less suds mileage. The weight average branching, described herein, has been found to provide improved low temperature stability, initial foam generation and suds longevity.

The weight average degree of branching for the alkyl sulfated anionic surfactant can be calculated using the following formula. The alky glycol sulfate anionic surfactant, and its precursors are excluded from the calculation.

Weight average degree of branching (%) = [(x1 * wt% branched alcohol 1 in alcohol 1 + x2 * wt% branched alcohol 2 in alcohol 2 + ....) / (x1 + x2 + ....)] * 100

where x1, x2, ... are the weight in grams of each alcohol in the total alcohol mixture of the alcohols which were used as starting material before (alkoxylation and) sulfation to produce the alkyl (alkoxy) sulfate anionic surfactant. In the weight average degree of branching calculation, the weight of the alkyl alcohol used to form the alkyl sulfate anionic surfactant which is not branched is included.

The weight average degree of branching and the distribution of branching can typically be obtained from the technical data sheet for the surfactant or constituent alkyl alcohol. Alternatively, the branching can also be determined through analytical methods known in the art, including capillary gas chromatography with flame ionization detection on medium polar capillary column, using hexane as the solvent. The weight average degree of branching and the distribution of branching is based on the starting alcohol used to produce the alkyl sulfate anionic surfactant.

Suitable counterions include alkali metal cation, earth alkali metal cation, alkanolammonium or ammonium or substituted ammonium, but preferably sodium, since the use of alkanolammonium or ammonium or substituted ammonium can lead to discoloration of the composition.

The alkyl alcohol can be derived from petrochemical feedstocks or on natural fats and oils. Three basic dominating commercial-scale processes are used to manufacture fatty alcohols: the Ziegler process and the OXO synthesis starting from petrochemical feedstocks, and the high-pressure hydrogenation of natural fatty acids and esters.

The Ziegler process (also called the Ziegler-Alfol synthesis) is a method for producing fatty alcohols from ethylene using an organoaluminium compound. The reaction produces linear primary alcohols with an even numbered carbon chain. The process uses an aluminum compound to oligomerize ethylene and allow the resulting alkyl group to be oxygenated. Suitable Ziegler processes are described in Klaus Noweck, Wolfgang Grafahrend (2006). "Fatty Alcohols". Ullmann's Encyclopedia of Industrial Chemistry. Weinheim: Wiley-VCH, and Zerong Wang "Ziegler Alcohol Synthesis (Ziegler Higher Alcohol Synthesis, Alfol Process, Ziegler-Alfol Process, Ziegler-Alfol Synthesis)" in Comprehensive Organic Name Reactions and Reagents, 2010, John Wiley & Sons, Inc.

Oxo alcohols are alcohols that are prepared through the addition of carbon monoxide (CO) and hydrogen (usually combined as synthesis gas) to an olefin to obtain an aldehyde using the hydroformylation reaction and then hydrogenating the aldehyde to obtain the alcohol. An intermediate step of adding two aldehydes together to obtain a larger aldehyde (the aldol condensation reaction) can precede the hydrogenation. Long chain oxo-alcohols are often prepared using alpha-olefins from the Shell higher olefin process, to give secondary alcohols such as isodecyl alcohol. Suitable OXO processes are described in Weber, Hermann; Falbe, Jurgen (April 1970). "Oxo Synthesis Technology". Industrial & Engineering Chemistry. 62 (4): 33-37, and Wagner, John D.; Lappin, George R.; Zietz, J. Richard (2000). "Alcohols, Higher Aliphatic, Synthetic Processes". Kirk-Othmer Encyclopedia of Chemical Technology.

High-pressure hydrogenation can be used with triglycerides, fatty acids or fatty acid esters as feedstock. Conversion of fatty acids into fatty alcohols by catalytic hydrogenation without preesterification typically requires corrosion-resistant materials of construction and acid-resistant catalysts. Required reaction temperatures are also higher, resulting in a higher hydrocarbon content. Therefore, the majority of fatty alcohol plants based on natural fats and oils use methyl esters as feedstock. These can be made either by esterification of fatty acids or by-transesterification of triglycerides. For catalytic high-pressure hydrogenation of methyl esters to fatty alcohols, several process options have been developed. The basic distinguishing feature is the catalyst application either in a fixed bed arrangement or suspended in the methyl ester feed. Suitable processes are described in Kreutzer, U.R. "Manufacture of fatty alcohols based on natural fats and oils." J Am Oil Chem Soc 61, 343-348 (1984).

Suitable examples of commercially available alkyl alcohols include, those derived from alcohols sold under the Neodol® brand-name by Shell, or the Lial®, Isalchem®, and Safol® brand-names by Sasol, or some of the natural alcohols produced by The Procter & Gamble Chemicals company. The alcohols (and alkoxylated alcohols) can be blended in order to achieve the desired mol fraction of C12 and C13 chains and the desired C13/C12 ratio, based on the relative fractions of C13 and C12 within the starting alcohols (as well as the desired degree of alkoxylation), as obtained from the technical data sheets from the suppliers or from analysis using methods known in the art.

The alkyl alcohol can comprise alkyl chains which are essentially linear or even fully linear, which are blended with the branched alcohol to achieve the desired degree of branching. Preferred sources of naturally derived alkyl chains include palm kernel and coconut derived alkyl chains, with palm kernel derived alkyl chains being more preferred. The naturally derived alkyl chain can be fractionated in order to provide the desired average alkyl chain length, as well as to adjust the alkyl chain length distribution. The C12 to C14 fraction is often referred to as the mid cut fraction within the naturally derived alkyl chains.

The alkyl alcohol can be fractionated, such as via distillation, in order to provide the desired "cut".

Sulfation step

In the sulfation step, the alcohol stream comprising the alkyl glycol and alkyl alcohol is sulfated to form an alkyl sulfuric acid stream. The alkyl sulfuric acid stream comprises a blend of alkyl glycol sulfuric acid and alkyl sulfuric acid.

Sulfation involves forming a carbon-oxygen-sulfur bond. The resultant alkyl glycol sulfuric acid and alkyl sulfuric acid in acid form are not hydrolytically stable. Unless neutralized, it decomposes to form sulfuric acid and other chemicals.

Sulfation of alcohols or alkoxylated alcohols into their equivalent sulfuric acids has been described extensively. More details of such a sulfation step has been described in "Sulf(on)ation Technology in the Detergent Industry" (W. Herman de Groot, Springer-Science+Business Media, B.V., 1991, ISBN 978-90-481-4088-6).

Various reagents can be used for the sulfation step, with sulfur trioxide ($SO_3$) being particularly preferred, at least partially due to its low cost. $SO_3$ is an aggressive electrophilic reagent that rapidly reacts with any organic compound containing an electron donor group. The resultant reaction is highly exothermic. Effective cooling of the reaction mass is essential because high temperatures promote side reactions that produce undesirable by-products. Also, precise control of the molar ratio of $SO_3$ to alkyl alcohol / alkyl glycol is essential because any excess $SO_3$, due to its reactive nature, contributes to side reactions and by-product formation. Therefore, commercial scale sulfation reactions require special equipment and instrumentation that allows tight control of the mole ratio of $SO_3$ to alkyl alcohol and rapid removal of the heat of reaction.

The problem of $SO_3$ reactivity has typically been solved by diluting and/or complexing the $SO_3$ to moderate the rate of reaction. Commercial diluting or complexing agents include ammonia (sulfamic acid), hydrochloric acid (chlorosulfuric acid), and dry air (air/$SO_3$ film sulfation). Control of the ratio of $SO_3$ to alkyl alcohol / alkyl glycol can be used to achieve improved product quality with use of any of these reagents.

Air/$SO_3$ film sulfation processes are typically carried out using a liquid-gas interface reactor, preferably a falling film reactor, such as an annular falling film reactor, such as a "Chemithon" reactor, or a multi-tube film reactor, such as the "Ballestra" reactor. In such processes, the $SO_3$ is first diluted with dry air. Such air/$SO_3$ sulfation processes are direct processes in which $SO_3$ gas is diluted with very dry air and reacted directly with the alkyl alcohol / alkyl glycol feedstock. The reaction of gaseous $SO_3$ with the alkyl alcohol / alkyl glycol is rapid and stoichiometric. Such processes are complicated by the possibility of side reactions, However, with tight process control, very high purity alkyl sulfated and alkyl glycol sulfate surfactants can be achieved.

The $SO_3$ can be provided by burning molten sulfur in an excess of oxygen to form $SO_2$, which is then catalytically oxidised to $SO_3$, for instance, at a temperature of from 400 °C to 470 °C. The oxygen can be supplied by air which has been pre-dried to remove the major part of water by condensation and subsequently drying with a desiccant until the air has a maximum dewpoint of -60°C, preferably < -70°C. Considering the exothermic nature of the sulfur burning reaction, the $SO_2$/air flow is cooled in an indirect air cooler, prior to converting the $SO_2$ to $SO_3$ through a catalytic oxidation. Catalytic oxidation is typically done using at least one, preferably from 3 to 4 catalytic beds. An example of such a catalytic converter includes a converter tower filled with 4 packed beds of $V_2O_5$ catalyst on a silica carrier. Considering the exothermic nature of the $SO_2$ to $SO_3$ oxidation, an intermediate cooling of the resulting process gas is executed between the various beds through indirect air coolers, such as vertical air cooled shell and tube heat exchangers. Despite the air pre-drying step there is still some sulfuric acid / oleum mist condensed which is removed through a demister, such as a Brinks filter, prior to the sulfation step. To ensure sufficient mist is removed the gas stream at this point in the process is cooled to at least 60°C, but most preferably the gas stream is cooled to a temperature of from 30 to 55 °C.

In air/$SO_3$ film sulfation processes, the sulfation step is typically carried out in a liquid-gas interface reactor, preferably a falling film reactor. Suitable falling film reactors include annular-gap falling film ("Chemithon") reactors, (multi) tubular ("Ballestra") reactors, and the like.

The blend of alkyl alcohols and alkyl glycols is converted through reaction with the $SO_3$ within the falling film reactor into alkyl sulfuric acid and alkyl glycol sulfuric acid. The sulfation is typically completed to a degree of sulfation of at least 95%, preferably at least 96%, more preferably at least 98%. Considering the exothermic nature of the sulfonation reaction, consequent cooling is again required after which the air/gas is separated from the liquid stream. Ideally the reaction mixture is maintained at the outlet of the reactor at a temperature of from 15 °C to 50 °C, preferably from 30 °C and 40°C.

The air/gas can be separated from the liquid using a liquid separator. Where the separation does not remove sufficient gas from the liquid mixture, an extra degassing step can be done, for instance, by using a "Fryma" spinning disc deaerator.

The exhaust gas typically comprises small amounts of non-converted $SO_2$, non-reacted $SO_3$ and some entrained organic acid. The organic aerosol and fine $SO_3$/$H_2SO_4$ droplets are typically separated from the exhaust gas flow in an electrostatic precipitator, and the gaseous $SO_2$ gasses are typically washed from the process air in a scrubber using a dilute caustic solution.

The sulfation can be completed to a degree of sulfation of at least 95%, preferably at least 96%, more preferably at least 98%. That is, the sulfation reaction results in a conversion of at least 95%, preferably at least 96%, more preferably at

least 98% by weight of the starting alkyl glycol and alkyl alcohol. A degree of sulfation of 100% is typically not achieved. As such, residual amounts of unsulphated alkyl glycol and alkyl alcohol are typically present. The sulfuric acid presence in the liquid stream after sulfation is typically less than 1%, preferably less than 0.75%, more preferably less than 0.5% by weight of the total liquid stream.

The unreacted alkyl glycol and alkyl alcohol levels after sulfation is preferably below 3%, more preferably below 2.5%, and most preferably below 2% by weight of the starting alkyl sulfuric acid and alkyl glycol sulfuric acid at the start of the neutralisation step. The amount of unreacted alkyl glycol and alkyl alcohol can be determined by GC analysis (after neutralisation of the alkyl sulfuric acid).

Neutralisation step

Considering the sensitivity of protonated alkyl sulfuric acid and alkyl glycol sulfuric acid to re-hydrolyse into the starting alkyl alcohols and alkyl glycols, a consequent neutralisation step is required. Without neutralisation, re-hydrolysis of the respective sulfuric acid leads to a decreased alkyl glycol sulfate and alkyl sulfated anionic surfactant content in the resultant concentrated surfactant blend, and also to an increased sulfuric acid content which results in discoloration of the concentrated surfactant blend and liquid detergent compositions made with the concentrated surfactant blend.

In the present process, a neutralising stream comprising at least one neutralising agent is provided. During and optionally further after the neutralisation step, the neutralising agent can be added at a level to provide the resultant concentrated surfactant blend with a pH of at least 7.0, or from 7.1 to 12, preferably from 7.3 to 9.5, more preferably from 7.5 to 9.0, measured as a 10% by weight solution of the concentrated surfactant blend in demineralized water, at 20°C.

Too low a pH can lead to re-hydrolysis of the alkyl glycol sulfate and alkyl sulfated anionic surfactant, while too high a pH typically complicates conversion of the concentrated surfactant blend into a finished detergent product composition, such as requiring a significant amount of acid to trim back the pH to the targeted finished product pH. In addition, too high a pH can result in the generation of 1,4-dioxanes over time, as well as higher amounts of neutralizing agent being added, increasing the salt level in the concentrated surfactant blend accordingly. Concentrated surfactant blends which comprise high salt levels are more challenging to formulate into detergent compositions, and result in a reduced low temperature stability as well as increased viscosity upon initial dissolution with water, slowing down product dissolution accordingly.

The neutralising agent is an alkali. Further neutralising agent can be added after the neutralisation step in order to adjust the pH to the desired level. Suitable alkali can be selected from the group consisting of sodium hydroxide potassium hydroxide, ammonia, monoethanolamine, di-ethanolamine, tri-ethanolamine, and mixtures thereof, with sodium hydroxide being most preferred. Alternatively, or in addition, other alkalis can be added as part of the neutralisation step. Particularly suitable other alkalis are alkalis that can be added to the resultant liquid detergent composition to improve performance. In particular, amines, especially cyclic polyamine having amine functionalities that helps cleaning, as described later.

The neutralisation step should be completed in less than 10 minutes, preferably less than 5 minutes and most preferably less than 2 minutes, after the sulfation step has been completed.

The neutralisation can take place in any suitable means, including in a batch reactor or by combining the alkyl sulfuric acid stream and neutralising stream using a high shear mixer. In preferred processes, a loop reactor can be used. A loop reactor is a continuous tube or pipe, typically stainless steel, which connects the outlet of a recirculation pump to its inlet. Reactants are fed into the loop where the neutralisation occurs, and the at least partially neutralised mixture is withdrawn from the loop.

As such, a loop reactor typically comprises a circulation pump, a homogenizer or high shear mixer, and a heat exchanger, due to the exothermic nature of the neutralisation reaction. Efficient mixing of the alkyl sulfuric acid stream and the neutralising stream results in instantaneous reaction and avoids undesired degradation reactions in isolated spots and pH drift occurring during the neutralisation step. As such high shear mixers are typically used considering the highly viscous nature of the resulting paste, especially at low shear rates.

As the neutralization reaction is exothermic, a heat exchanger, such as a plate and frame heat exchanger, can be used to continuously cool the mixture to achieve a temperature of less than 70°C, preferably less than 60°C and most preferably in a range of 20 to 40°C for the alkyl sulfate stream after neutralisation.

Water or organic solvents can be added either to control the viscosity during the neutralisation step or to improve homogenisation. Suitable organic solvents include C1-C4 alcohols, particularly ethanol. Such organic solvents can be added as a separate stream during the neutralisation step, or as part of the alkyl alcohol stream, or as part of the neutralisation stream. Water can be added as a separate stream during the neutralisation step, or as part of the neutralisation stream.

Water can be added at a level such that the resultant concentrated surfactant blend comprises water at a level of from 20% to 50%, preferably from 25% to 45%, more preferably from 30% to 40% by weight of the concentrated surfactant blend. The water level can be measured using any suitable means, such as via Karl Fisher Titration,

The neutralisation stream can comprise water at a level of from 35% to 80%, preferably from 45% to 75%, more preferably from 55% to 65% by weight of the neutralisation stream.

The neutralising stream can comprise other optional ingredients, such as nonionic surfactant, polymers, and peroxides, such as those described later, and mixtures thereof.

After the neutralization step the resulting neutralized surfactant paste can be transported through a transfer pump into a storage tank.

The dioxane level in the concentrated surfactant blends formed by the process of the present invention can be less than 40 ppm, preferably less than 30 ppm, most preferably less than 15 ppm after the neutralization step, expressed on a 100% anionic surfactant active basis measured immediately after the neutralizing step. The 1,4-dioxane level is measured within one hour of the completion of the neutralization step.

Optional further steps

Buffering

A buffering surfactant can be added at any suitable point before or during the neutralising step. The buffering surfactant is preferably added during the neutralization step, either as a separate buffer stream, but preferably as part of the neutralisation stream. Alternatively, the buffering surfactant can be added before the neutralisation step. The alkyl sulfuric acid, alkyl glycol sulfuric acid and the buffering surfactant can be combined in a weight ratio of the combined alkyl sulfuric acid / alkyl glycol sulfuric acid to the buffering surfactant of from 10:1 to 1:1, preferably from 8:1 to 2:1, more preferably from 6:1 to 3:1.

Suitable buffering surfactants can be an amphoteric surfactant, zwitterionic surfactant, or mixtures thereof. Suitable buffering surfactants can be selected from the group consisting of: amine oxide surfactant, betaine surfactant, and mixtures thereof, preferably amine oxide surfactant, more preferably C10-16 dimethyl amine oxide surfactant. C12-14 dimethyl amine oxide (lauryl dimethyl amine oxide) is particularly preferred. Such buffering surfactants also contribute to the performance of the resultant liquid hand dishwashing detergent. In addition, the buffering surfactant results in the concentrated surfactant blends, formed by the processes described herein, having reduced viscosity, especially when amine oxide surfactant is used as the buffering surfactant.

The buffering surfactant reduces the pH variation and pH drift when small amounts of acid are added or formed, for instance, through hydrolysis of the alkyl sulfated / alkyl glycol sulfate surfactant, or even from absorption of carbon dioxide from the air. As a result, the resultant concentrated surfactant blend can be stored at lower pH, resulting in less formation of 1,4-dioxanes and less salt being present in detergent compositions comprising the concentrated surfactant blend. As such, when the present process utilises a buffering surfactant, a lower pH for the concentrated surfactant blend can be used. As a result, lower levels of 1,4-dioxane are present in the concentrated surfactant blend. Moreover, the resultant alkyl sulfated / alkyl glycol sulfate containing concentrated surfactant blends exhibit lower rates of increase in the 1,4-dioxane by-product level upon ageing.

Therefore, when a buffering surfactant is added in the present process, the neutralising agent can be added at a level to provide the resultant concentrated surfactant blend with a pH of from 7.1 to 10, more preferably from 7.3 to 9.5, most preferably from 7.5 to 9.0, measured as a 10% by weight solution of the concentrated surfactant blend in demineralized water, at 20°C.

When added, the buffering surfactant can be added at a level to provide the resultant concentrated surfactant blend with a reserve alkalinity of greater than 0.02 when measured as a 10% by weight solution of the concentrated surfactant blend in demineralized water at 20°C, using the method as described herein, with the resultant concentrated surfactant blend having a pH of from 7.1 to 10, when measured as a 10% by weight solution of the concentrated surfactant blend in demineralized water at 20°C. Since the buffering surfactant reduces pH fluctuations and reduces or even prevents pH drift in the resultant concentrated surfactant blend, a lower pH can be used while still avoiding hydrolysis of the alkyl sulfated / alkyl glycol sulfate anionic surfactant blend. Since the concentrated surfactant blend can be kept at a lower pH, the formation of 1,4-dioxane is further reduced.

The buffering capacity is the ability to neutralize the pH and the resistance to change in it due to the small acidic or basic inputs or discharges. When a system is poorly buffered, the addition of even small amounts of an acid or a base will noticeably alter its pH, but when a system is well buffered, the same addition barely modifies its pH.

Reserve alkalinity is a measure often used industrially to indicate the amount of alkaline components present in the product. For some compositions, such as the concentrated surfactant blends disclosed herein, it is more important to know the reserve alkalinity of the blend rather than the buffer capacity, because the reserve alkalinity provides a measure of the capacity of the blend to neutralise any acid that is present, added, or formed *in-situ,* and hence maintain an alkaline pH.

The buffering surfactant can be added at a level such that the resultant concentrated surfactant blend comprises the buffering surfactant at a level of from 1.0% to 25% preferably from 5.0% to 20%, more preferably from 10% to 15% by

weight of the concentrated surfactant blend.

Suitable amine oxide surfactants can be linear or branched, though linear are preferred. Suitable linear amine oxides are typically water-soluble, and characterized by the formula R1 - N(R2)(R3) O wherein R1 is a C8-18 alkyl, and the R2 and R3 moieties are selected from the group consisting of C1-3 alkyl groups, C1-3 hydroxyalkyl groups, and mixtures thereof. For instance, R2 and R3 can be selected from the group consisting of: methyl, ethyl, propyl, isopropyl, 2-hydroxethyl, 2-hydroxypropyl and 3-hydroxypropyl, and mixtures thereof, though methyl is preferred for one or both of R2 and R3. The linear amine oxide surfactants in particular may include linear C10-C18 alkyl dimethyl amine oxides and linear C8-C12 alkoxy ethyl dihydroxy ethyl amine oxides.

Preferably, the amine oxide surfactant is selected from the group consisting of: alkyl dimethyl amine oxide, alkyl amido propyl dimethyl amine oxide, and mixtures thereof. Alkyl dimethyl amine oxides are particularly preferred, such as C8-18 alkyl dimethyl amine oxides, or C10-16 alkyl dimethyl amine oxides (such as coco dimethyl amine oxide). Suitable alkyl dimethyl amine oxides include C10 alkyl dimethyl amine oxide surfactant, C10-12 alkyl dimethyl amine oxide surfactant, C12-C14 alkyl dimethyl amine oxide surfactant, and mixtures thereof. C12-C14 alkyl dimethyl amine oxide, C12-14 alkyl amido propyl amine oxide, and mixtures thereof are particularly preferred.

Alternative suitable amine oxide surfactants include mid-branched amine oxide surfactants. As used herein, "mid-branched" means that the amine oxide has one alkyl moiety having n1 carbon atoms with one alkyl branch on the alkyl moiety having n2 carbon atoms. The alkyl branch is located on the $\alpha$ carbon from the nitrogen on the alkyl moiety. This type of branching for the amine oxide is also known in the art as an internal amine oxide. The total sum of n1 and n2 can be from 10 to 24 carbon atoms, preferably from 12 to 20, and more preferably from 10 to 16. The number of carbon atoms for the one alkyl moiety (n1) is preferably the same or similar to the number of carbon atoms as the one alkyl branch (n2) such that the one alkyl moiety and the one alkyl branch are symmetric. As used herein "symmetric" means that | n1 - n2 | is less than or equal to 5, preferably 4, most preferably from 0 to 4 carbon atoms in at least 50 wt%, more preferably at least 75 wt% to 100 wt% of the mid-branched amine oxides for use herein. The amine oxide further comprises two moieties, independently selected from a C1-3 alkyl, a C1-3 hydroxyalkyl group, or a polyethylene oxide group containing an average of from about 1 to about 3 ethylene oxide groups. Preferably, the two moieties are selected from a C1-3 alkyl, more preferably both are selected as C1 alkyl.

Alternatively, the amine oxide surfactant can be a mixture of amine oxides comprising a mixture of low-cut amine oxide and mid-cut amine oxide. The amine oxide of the composition of the invention can then comprises:

a) from about 10% to about 45% by weight of the amine oxide of low-cut amine oxide of formula R1R2R3AO wherein R1 and R2 are independently selected from hydrogen, C1-C4 alkyls or mixtures thereof, and R3 is selected from C10 alkyls and mixtures thereof; and

b) from 55% to 90% by weight of the amine oxide of mid-cut amine oxide of formula R4R5R6AO wherein R4 and R5 are independently selected from hydrogen, C1-C4 alkyls or mixtures thereof, and R6 is selected from C12-C16 alkyls or mixtures thereof

In a preferred low-cut amine oxide for use herein R3 is n-decyl, with preferably both R1 and R2 being methyl. In the mid-cut amine oxide of formula R4R5R6AO, R4 and R5 are preferably both methyl.

Preferably, the amine oxide comprises less than about 5%, more preferably less than 3%, by weight of the amine oxide of an amine oxide of formula R7R8R9AO wherein R7 and R8 are selected from hydrogen, C1-C4 alkyls and mixtures thereof and wherein R9 is selected from C8 alkyls and mixtures thereof. Limiting the amount of amine oxides of formula R7R8R9AO improves both physical stability and suds mileage.

Suitable zwitterionic surfactants include betaine surfactants. Such betaine surfactants includes alkyl betaines, alkylamidobetaine, amidazoliniumbetaine, sulfobetaine (INCI Sultaines) as well as the phosphobetaine, and preferably meets formula (I):

$$R^1\text{-}[CO\text{-}X(CH_2)_n]_x\text{-}N^+(R^2)(R_3)\text{-}(CH_2)_m\text{-}[CH(OH)\text{-}CH_2]_y\text{-}Y^-$$

Wherein in formula (I),

R1 is selected from the group consisting of: a saturated or unsaturated C6-22 alkyl residue, preferably C8-18 alkyl residue, more preferably a saturated C10-16 alkyl residue, most preferably a saturated C12-14 alkyl residue;
X is selected from the group consisting of: NH, NR4 wherein R4 is a C1-4 alkyl residue, O, and S,
n is an integer from 1 to 10, preferably 2 to 5, more preferably 3,
x is 0 or 1, preferably 1,
R2 and R3 are independently selected from the group consisting of: a C1-4 alkyl residue, hydroxy substituted such as a hydroxyethyl, and mixtures thereof, preferably both R2 and R3 are methyl,
m is an integer from 1 to 4, preferably 1, 2 or 3,

y is 0 or 1, and
Y is selected from the group consisting of: COO, SO3, OPO(ORS)O or P(O)(OR5)O, wherein R5 is H or a C1-4 alkyl residue.

Preferred betaines are the alkyl betaines of formula (Ia), the alkyl amido propyl betaine of formula (Ib), the sulfobetaine of formula (Ic) and the amido sulfobetaine of formula (Id):

$$R^1\text{-}N^+(CH_3)_2\text{-}CH_2COO^- \quad \text{(Ia)}$$

$$R^1\text{-}CO\text{-}NH\text{-}(CH_2)_3\text{-}N^+(CH_3)_2\text{-}CH_2COO^- \quad \text{(Ib)}$$

$$R^1\text{-}N^+(CH_3)_2\text{-}CH_2CH(OH)CH_2SO_3^- \quad \text{(Ic)}$$

$$R^1\text{-}CO\text{-}NH\text{-}(CH_2)_3\text{-}N^+(CH_3)_2\text{-}CH_2CH(OH)CH_2SO_3^- \quad \text{(Id)}$$

in which R1 has the same meaning as in formula (I). Particularly preferred are the carbobetaines [i.e. wherein Y-=COO- in formula (I)] of formulae (Ia) and (Ib), more preferred are the alkylamidobetaine of formula (Ib).

Suitable betaines can be selected from the group consisting or [designated in accordance with INCI]: capryl/-capramidopropyl betaine, cetyl betaine, cetyl amidopropyl betaine, cocamidoethyl betaine, cocamidopropyl betaine, cocobetaines, decyl betaine, decyl amidopropyl betaine, hydrogenated tallow betaine / amidopropyl betaine, isostearamidopropyl betaine, lauramidopropyl betaine, lauryl betaine, myristyl amidopropyl betaine, myristyl betaine, oleamidopropyl betaine, oleyl betaine, palmamidopropyl betaine, palmitamidopropyl betaine, palm-kernelamidopropyl betaine, stearamidopropyl betaine, stearyl betaine, tallowamidopropyl betaine, tallow betaine, undecylenamidopropyl betaine, undecyl betaine, and mixtures thereof. Preferred betaines are selected from the group consisting of: cocamidopropyl betaine, cocobetaines, lauramidopropyl betaine, lauryl betaine, myristyl amidopropyl betaine, myristyl betaine, and mixtures thereof. Cocamidopropyl betaine is particularly preferred.

The buffering surfactant can be added in a stream which further comprises peroxide, especially where amine oxide is used as a buffering surfactant. The peroxide can be present such that the stream comprising the buffering surfactant has a residual peroxide level of from 5.0 ppm to 300 ppm, preferably from 40 ppm to 80 ppm for every one part by weight of buffering surfactant. It is believed that the presence of peroxide further limits the formation and growth of 1,4-dioxane within the concentrated surfactant blend formed by the present process, when the concentrated surfactant blend comprises ethoxylated alkyl sulfate surfactant.

The resultant concentrated surfactant blend can comprise from 1.0 wt% to 25 wt%, preferably from 5.0 wt% to 20 wt%, more preferably from 10 wt% to 15 wt% by weight of the blend of the buffering surfactant. When present, the buffering surfactant buffers the pH of the concentrated surfactant blend without requiring additional salts to be introduced into the blend. As such, the concentrated surfactant blend resists changes in pH due to the addition or formation of an acid (or base). The resultant buffering reduces the risk of hydrolysis of the alkyl sulfated / alkyl glycol sulfate surfactant due to lowering pH, for example, upon ageing in contact with air, Re-hydrolysis would result in lowering the alkyl sulfated / alkyl glycol sulfate concentration but also resulting in the formation of by-products which cause browning of the concentrated surfactant blend (such as $HSO_4^-$). Moreover, without the buffering, a higher pH would be required for the concentrated surfactant blend, in order to avoid the pH dropping over time to a level that hydrolysis of the alkyl sulfated / alkyl glycol sulfate surfactant occurs too rapidly. The need to add additional alkali results in a higher ionic strength for unbuffered concentrated surfactant blends. This leads to reduced processability of the concentrated surfactant blend, as well as reduced dispersibility upon dilution, lower viscosity, as well as reduced foaming and suds milage for compositions comprising such unbuffered concentrated surfactant blends.

Buffers for a given application must be effective at the desired pH, and must also provide sufficient buffer capacity to maintain the desired pH as needed. In order to inhibit hydrolysis and minimise the formation of 1,4-dioxanes, the resultant concentrated surfactant blend, formed by the processes described herein, preferably have a buffer region of from a pH of 7.0 to 7.8.

A measure of the efficacy of the buffering system is provided by the reserve alkalinity. The reserve alkalinity is essentially the ability of the composition to neutralise acidic residues that are formed in the composition, such as by the hydrolysis of the alkyl sulfated / alkyl glycol sulfate surfactant. The buffering surfactant is added at a level to provide the resultant concentrated surfactant blend with a reserve alkalinity of greater than 0.02, preferably from 0.04 to 0.50, more preferably from 0.06 to 0.30 when measured as a 10% by weight solution of the concentrated surfactant blend in demineralized water at 20°C, using the method described herein.

The combined alkyl sulfated / alkyl glycol sulfate anionic surfactant and the buffering surfactant can be present in the concentrated surfactant blend in a weight ratio of from 10:1 to 1:1, preferably from 8:1 to 2:1, more preferably from 6:1 to 3:1.

1,4-dioxane removal

The level of 1,4-dioxane can be further reduced through stripping means, such as steam stripping. If used, a multi-step stripping process is typically utilized, usually involving 2 to 3 stripping steps. Such strippers are known in the art, and include steam strippers, for example, the Dioxane Removal System (DRS) provided by Chemithon. The concentrated surfactant blend is fed directly to the 1,4-dioxane stripping unit directly after the neutralisation step. The blend is injected into the top of a single-stage stripper. Steam is also injected into the top of the stripper at a rate proportional to the amount of 1,4-dioxane being stripped from the blend. Typically, 0.5 kilograms of steam are required per kilogram of neutralised paste to achieve an 8 to 1 reduction. Subsequently, the resultant mixture is passed to a separation tank. When the steam and 1,4-dioxane leave the separation tank through a vacuum system, the condensate and 1,4-dioxane are recovered. The stripping unit is typically jacketed to maintain the temperature in the desired range and the operating pressure is carefully controlled to avoid either concentrating or diluting the paste. Tempered water (hot water that has been cooled by adding cold water with a mixing device so that the water temperature is between 35 °C and 40 °C) can also be added to the stripper shell when stripping 1,4-dioxane.

Such strippers can reduce the level of 1,4-dioxane by-product by a factor of up to 8 for each stripping step. Hence, further reductions in 1,4-dioxane by-product can be achieved using a two stage, or even three stage stripping process. Since the final level of 1,4-dioxane in the concentrated surfactant blend is lower after stripping when the level of the dioxane by-product is lower in the blend before stripping, it is preferred to minimize the level of 1,4-dioxane in the concentrated surfactant blend, even if subsequent dioxane stripping is envisioned. The resultant concentrated surfactant blend undergoes a subsequent 1,4-dioxane stripping step, wherein the 1,4-dioxane level is further reduced to less than 10 ppm, preferably less than 5.0 ppm, most preferably less than 1.0 ppm, expressed on a 100% anionic surfactant active basis. The 1,4-dioxane level is measured within one hour of the completion of the stripping step.

Other steps:

If desired, adjunct ingredients as described below can also be added prior to, during or after neutralisation, for instance, to simplify making of the final liquid detergent composition. Other optional ingredients include water, organic solvents, pH modifier, further surfactants, polymers, amines, preservatives, and mixtures thereof. Suitable further surfactants include further anionic surfactants, nonionic surfactants, and mixtures thereof.

Concentrated surfactant blend

The resultant concentrated surfactant blend can comprise anionic surfactant at a level of from 20% to 80%, preferably from 20% to 40% or from 65% to 70%, most preferably from 20% to 30% by weight of the concentrated surfactant blend.

The concentrated anionic surfactant blend comprises an anionic surfactant, wherein the anionic surfactant comprises alkyl glycol sulfate anionic surfactant and alkyl sulfated anionic surfactant. The concentrated anionic surfactant blend can comprise further surfactants, such as further anionic surfactant.

The concentrated anionic surfactant blend can comprise at least 70%, more preferably at least 85%, most preferably 100% by weight of the anionic surfactant of the alkyl glycol sulfate anionic surfactant and the alkyl sulfated anionic surfactant. In preferred compositions, the anionic surfactant in the concentrated anionic surfactant blend consists of alkyl glycol sulfate anionic surfactant and the alkyl sulfated anionic surfactant. The alkyl glycol sulfate anionic surfactant and the alkyl sulfated anionic surfactant are preferably present in the blend at a weight ratio of from 1:10 to 2:1, preferably from 1:7 to 1:1, and most preferably from 1:5 to 1:2.

Without wishing to be bound by theory, it is believed that a mixture provides a surfactant packing which balances grease cleaning and suds mileage performance, especially in presence of greasy-particulate soils, low temperature stability and demonstrating a minimum impact on the targeted finished product viscosity, as well as a lower viscosity and improved low temperature stability of the concentrated surfactant blend itself.

Alkyl glycol sulfate anionic surfactant

Preferably the alkyl glycol sulfate anionic surfactant in the concentrated anionic surfactant blend is at a level of from 5.0 % to 50 % preferably from 10 % to 40 %, more preferably from 15 % to 30 % by weight of the concentrated anionic surfactant blend.

Alkyl ethoxylated sulfate anionic surfactants are typically formed through processes whereby an alkyl alcohol is first ethoxylated, before being sulfated. In contrast, alkyl glycol sulfate anionic surfactants are typically prepared by first reacting an ethylene glycol monomer or oligomer with an alkene, before sulfation. Since the resultant sulfate surfactant is not formed using an ethoxylation step, it is technically incorrect to refer to them as alkyl ethoxylated surfactants. As such,

and since they are typically formed by reacting an alkene with glycol (aliphatic diol), they are referred to here as alkyl glycol sulfate anionic surfactants, for example alkyl ethylene glycol sulfate (AEGS). Such processes, for example as described in WO202464645A, result in a much narrower distribution of the glycol groups in the resultant alkyl glycol sulfate anionic surfactants, and hence enable significant reductions in the presence and formation of dioxanes within the detergent formulae containing them. This is because little or no 1,4-dioxane is formed during the production of alkyl ethylene glycol sulfates. Moreover, by limiting the amount of alkyl ethylene glycol sulfate having two or more glycol units present, production of 1,4-dioxane through the degradation of the surfactant is substantially reduced.

The alkyl glycol sulfate anionic surfactant has the formula (II):

$$R1CH(R2)(OCH_2CH_2)_nOSO_3^-M^+ \qquad (II)$$

wherein R2, R2, and n are as described for formula (I).

$M^+$ of structure (I) is a counterion, preferably $M^+$ is an alkali metal counterion or ammonium, ethanolamine, isopropanolamine, triethylamine, triethanolamine, N-methyl diethanolamine, N,N-dimethyl ethanolamine, N,N-dimethyl propanolamine, and combinations thereof, more preferably $Na^+$, $K^+$, $Mg^{2+}$, or ethanolamine, most preferably $Na^+$. It will be understood that different surfactant molecules may have different materials for $M^+$.

The alkyl glycol sulfate anionic surfactants described herein comprise an extremely narrow distribution of ethylene glycol units in the alkyl glycol sulfate anionic surfactant, typically from 1 to 3 ethylene glycol units per molecule of alkyl glycol sulfate anionic surfactant, or even 1 ethylene glycol unit per molecule of alkyl glycol sulfate anionic surfactant.

Alkyl sulfated anionic surfactant

The concentrated surfactant blend further comprises alkyl sulfated anionic surfactant, formed from the sulfation and subsequent neutralisation of the (optionally alkoxylated) alkyl alcohol in the alcohol stream. While less preferred, further alkyl sulfated anionic surfactant can be added to arrive at the desired composition for the resultant concentrated surfactant blend. However, preferably, no further alkyl sulfated anionic surfactant is added, beyond that formed by the sulfation and subsequent neutralisation of the alkyl alcohol in the alcohol stream. The concentrated surfactant blend can comprise at least 25%, preferably from 30% to 90%, more preferably from 65% to 85% by weight of the anionic surfactant of alkyl sulfated anionic surfactant.

Additional surfactant

The concentrated surfactant blend can comprise additional anionic surfactant such as those selected from the group consisting of: alkyl (benzene) sulfonate surfactant, alkyl sulfosuccinate and dialkyl sulfosuccinate ester surfactants, and mixtures thereof. However, in preferred processes, the amount of such further anionic surfactants is kept low. In more preferred processes, no further anionic surfactant is added. Therefore, the concentrated surfactant blend can comprise at least 70%, preferably at least 85%, more preferably 100% by weight of the anionic surfactant of the alkyl sulfated anionic surfactant and alkyl glycol sulfate anionic surfactant. The concentrated surfactant blend is preferably free of fatty acid or salt thereof, since such fatty acids impede the generation of suds.

Anionic alkyl sulfonate or sulfonic acid surfactants suitable for use herein include the acid and salt forms of alkylbenzene sulfonates, alkyl ester sulfonates, primary and secondary alkane sulfonates such as paraffin sulfonates, alfa or internal olefin sulfonates, alkyl sulfonated (poly)carboxylic acids, and mixtures thereof. Suitable anionic sulfonate or sulfonic acid surfactants include: C5-C20 alkylbenzene sulfonates, more preferably C10-C16 alkylbenzene sulfonates, more preferably C11-C13 alkylbenzene sulfonates, C5-C20 alkyl ester sulfonates especially C5-C20 methyl ester sulfonates, C6-C22 primary or secondary alkane sulfonates, C5-C20 sulfonated (poly)carboxylic acids, and any mixtures thereof, but preferably C11-C13 alkylbenzene sulfonates. The aforementioned surfactants can vary widely in their 2-phenyl isomer content. Compared with sulfonation of alpha olefins, the sulfonation of internal olefins can occur at any position since the double bond is randomly positioned, which leads to the position of hydrophilic sulfonate and hydroxyl groups of IOS in the middle of the alkyl chain, resulting in a variety of twin-tailed branching structures. Alkane sulfonates include paraffin sulfonates and other secondary alkane sulfonate (such as Hostapur SAS60 from Clariant).

Alkyl sulfosuccinate and dialkyl sulfosuccinate esters are organic compounds with the formula MO3SCH(CO2R')CH2CO2R where R and R' can be H or alkyl groups, and M is a counter-ion such as sodium (Na). Alkyl sulfosuccinate and dialkyl sulfosuccinate ester surfactants can be alkoxylated or non-alkoxylated, preferably non-alkoxylated. The surfactant system may comprise further anionic surfactant. However, the composition preferably comprises less than 30%, preferably less than 15%, more preferably less than 10% by weight of the surfactant system of further anionic surfactant. Most preferably, the surfactant system comprises no further anionic surfactant, preferably no other anionic surfactant than alkyl sulfated anionic surfactant and the alkyl glycol sulfate anionic surfactant.

Suitable further surfactants include nonionic surfactants. The concentrated surfactant blend can further comprise a nonionic surfactant. Suitable nonionic surfactants include alkoxylated alcohol nonionic surfactants, alkyl polyglucoside nonionic surfactants, and mixtures thereof.

The concentrated surfactant blend can comprise from 1% to 25%, preferably from 1.25% to 20%, more preferably from 1.5% to 15%, most preferably from 1.5% to 5%, by weight of the total surfactant within the concentrated surfactant blend, of an alkoxylated alcohol non-ionic surfactant.

Preferably, the alkoxylated alcohol non-ionic surfactant is a linear or branched, primary or secondary alkyl alkoxylated non-ionic surfactant, preferably an alkyl ethoxylated non-ionic surfactant, preferably comprising on average from 9 to 15, preferably from 10 to 14 carbon atoms in its alkyl chain and on average from 5 to 12, preferably from 6 to 10, most preferably from 7 to 8, units of ethylene oxide per mole of alcohol.

The concentrated surfactant blend can comprise alkyl polyglucoside ("APG") surfactant. The addition of alkyl polyglucoside surfactants has been found to improve sudsing beyond that of comparative nonionic surfactants such as alkyl ethoxylated nonionic surfactants. If present, the alkyl polyglucoside can be present in the concentrated surfactant blend at a level of from 0.5% to 20%, preferably from 0.75% to 15%, more preferably from 1% to 10%, most preferably from 1% to 5% by weight of the total surfactant within the concentrated surfactant blend. Preferably the alkyl polyglucoside surfactant is a C8-C16 alkyl polyglucoside surfactant, preferably a C8-C14 alkyl polyglucoside surfactant. The alkyl polyglucoside preferably has an average degree of polymerization of between 0.1 and 3, more preferably between 0.5 and 2.5, even more preferably between 1 and 2. Most preferably, the alkyl polyglucoside surfactant has an average alkyl carbon chain length between 10 and 16, preferably between 10 and 14, most preferably between 12 and 14, with an average degree of polymerization of between 0.5 and 2.5 preferably between 1 and 2, most preferably between 1.2 and 1.6.

C8-C16 alkyl polyglucosides are commercially available from several suppliers (e.g., Simusol® surfactants from Seppic Corporation; and Glucopon® 600 CSUP, Glucopon® 650 EC, Glucopon® 600 CSUP/MB, and Glucopon® 650 EC/MB, from BASF Corporation).

Alternatively, the concentrated surfactant blend is free of other surfactants than anionic surfactant, most preferably wherein the anionic surfactant consists of the alkyl sulfated anionic surfactant and the alkyl glycol sulfate anionic surfactant.

The concentrated surfactant blend can be Newtonian or non-Newtonian, preferably Newtonian. The surfactant blend can have a viscosity of from 5,000 mPa•s to 25,000 mPa•s, preferably from 7,500 mPa•s to 20,000 mPa•s, most preferably from 10,000 mPa•s to 15,000 mPa•s, measured at a shear rate of 10 $s^{-1}$ and a temperature of 20° C.

The concentrated surfactant blend can have a flow index of from 0.1 to 1.0, preferably from 0.2 to 0.5, more preferably from 0.2 to 0.4. The concentrated surfactant blend can have a yield stress of from 5.0 Pa to 30 Pa, preferably from 10 Pa to 25 Pa, more preferably from 15 Pa to 20 Pa. The aforementioned flow index and yield stress result in improved processibility of the concentrated surfactant blend.

The melting point of the concentrated surfactant blend is preferably less than 20 °C, more preferably less than 15 °C, most preferably less than 10 °C so that the concentrated surfactant blend can be stored at ambient temperatures and not require heating in order to process into a detergent composition. The melting point can be measured using Differential Scanning Calorimetry" (DSC).

The concentrated surfactant blend can have a density in the range from 500 $kg/m^3$ to 1,500 $kg/m^3$, preferably from 750 $kg/m^3$ to 1,250 $kg/m^3$, more preferably from 1,000 $kg/m^3$ to 1,100 $kg/m^3$, measured at a temperature of 20 °C. The density can be measured using any suitable means, such as using a pycnometer.

Additional ingredients

The concentrated surfactant blends produced by the processes described herein are typically more readily flowable. As such, little or no nonaqueous solvent is typically added to the concentrated surfactant blend. As such, the concentrated surfactant blend comprises less than 15%, preferably less than 10%, more preferably less than 5% by weight or can even be essentially free or free of solvent. Suitable solvents can be selected from the group consisting of: alcohols, polyols, glycols, glycol ethers, and mixtures thereof. Preferably the non-aqueous solvent is selected from alcohols, most preferably is ethanol.

Residual amounts of other ingredients can be present, such as secondary alcohols, secondary alcohol sulfates, unreacted or partially reacted alpha olefin, unreacted ethylene glycol, and unsulfated alkyl glycol. Such residual amounts of other ingredients are typically present at a level of less than 25%, preferably less then 15%, more preferably less than 5% by weight of the surfactant blend.

Liquid hand dishwashing composition:

The concentrated surfactant blends, as described herein can be used to make liquid detergent compositions, especially liquid hand dishwashing detergent composition.

The term "dishware" and "dish" as used herein includes cookware and tableware made from, by non-limiting examples, ceramic, china, metal, glass, plastic (e.g., polyethylene, polypropylene, polystyrene, etc.) and wood.

The cleaning composition is a liquid cleaning composition, preferably a liquid hand dishwashing cleaning composition, and hence is in liquid form. The liquid cleaning composition is preferably an aqueous cleaning composition. As such, the composition can comprise from 50% to 85%, preferably from 50% to 75%, by weight of the total composition of water.

The liquid cleaning composition has a pH greater than 6.0, or a pH of from 6.0 to 12.0, preferably from 7.0 to 11.0, more preferably from 8.0 to 10.0, measured as a 10% aqueous solution in demineralized water at 20 degrees °C.

When the pH exceeds a pH of 7.0, the reserve alkalinity can be from 0.1 to 1.0, more preferably from 0.1 to 0.5. Reserve alkalinity is herein expressed as grams of NaOH/100 ml of composition required to titrate product from a pH 7.0 to the pH of the finished composition. This pH and reserve alkalinity further contribute to the cleaning of tough food soils.

The liquid cleaning composition of the present invention can be Newtonian or non-Newtonian, preferably Newtonian. Preferably, the composition has a viscosity of from 10 mPa•s to 10,000 mPa•s, preferably from 100 mPa•s to 5,000 mPa•s, more preferably from 300 mPa•s to 2,000 mPa•s, or most preferably from 500 mPa•s to 1,500 mPa•s, alternatively combinations thereof. The viscosity is measured at 20°C with a Brookfield RT Viscometer using spindle 31 with the RPM of the viscometer adjusted to achieve a torque of between 40% and 60%.

The liquid cleaning composition can comprise the concentrated surfactant blend and optionally additional surfactant, such that the composition comprises from 5.0% to 50%, preferably from 6.0% to 40%, most preferably from 15% to 35%, by weight of the total composition of a surfactant system.

The liquid hand dishwashing detergent composition comprises anionic surfactant that is comprised in the concentrated surfactant blend used to make the detergent composition. Preferably, the liquid hand dishwashing detergent composition does not comprise any further anionic surfactant.

The liquid hand dishwashing detergent composition can comprise a co-surfactant. The co-surfactant can be the optional buffering surfactant, when comprised in the concentrated surfactant blend used to make the detergent composition. Alternatively, or in addition, co-surfactant can be added, in addition to any co-surfactant comprised in the concentrated surfactant blend. The co-surfactant can include one or more amphoteric or zwitterionic surfactant, added to the detergent composition, in addition to the concentrated surfactant blend, described herein. This can also include adding an additional amount of the buffering surfactant used already in the concentrated surfactant blend in order to further increase its level inside the liquid detergent composition. The liquid hand dishwashing detergent composition can comprise nonionic surfactant. Suitable nonionic surfactants include alkoxylated alcohol nonionic surfactants, alkyl polyglucoside nonionic surfactants, and mixtures thereof, as described earlier. Such nonionic surfactant can be added as part of the concentrated surfactant blend used to make the detergent composition, or can be added separately and in addition to the concentrated surfactant blend, or both.

The composition can comprise further ingredients such as those selected from: amphiphilic alkoxylated polyalkyleneimines, cyclic polyamines, triblock copolymers, inorganic mono-, di- or trivalent salts, hydrotropes, organic solvents, other adjunct ingredients such as those described herein, and mixtures thereof. Such ingredients can be added as part of the surfactant blend, or separately added, in addition to the surfactant blend.

Amphiphilic alkoxylated polyalkyleneimine:

The composition of the present invention may further comprise from 0.05% to 2%, preferably from 0.07% to 1% by weight of the total composition of an amphiphilic polymer. Suitable amphiphilic polymers can be selected from the group consisting of: amphiphilic alkoxylated polyalkyleneimine and mixtures thereof. The amphiphilic alkoxylated polyalkyleneimine polymer has been found to improve grease removal.

A preferred amphiphilic alkoxylated polyethyleneimine polymer has the general structure of formula (I):

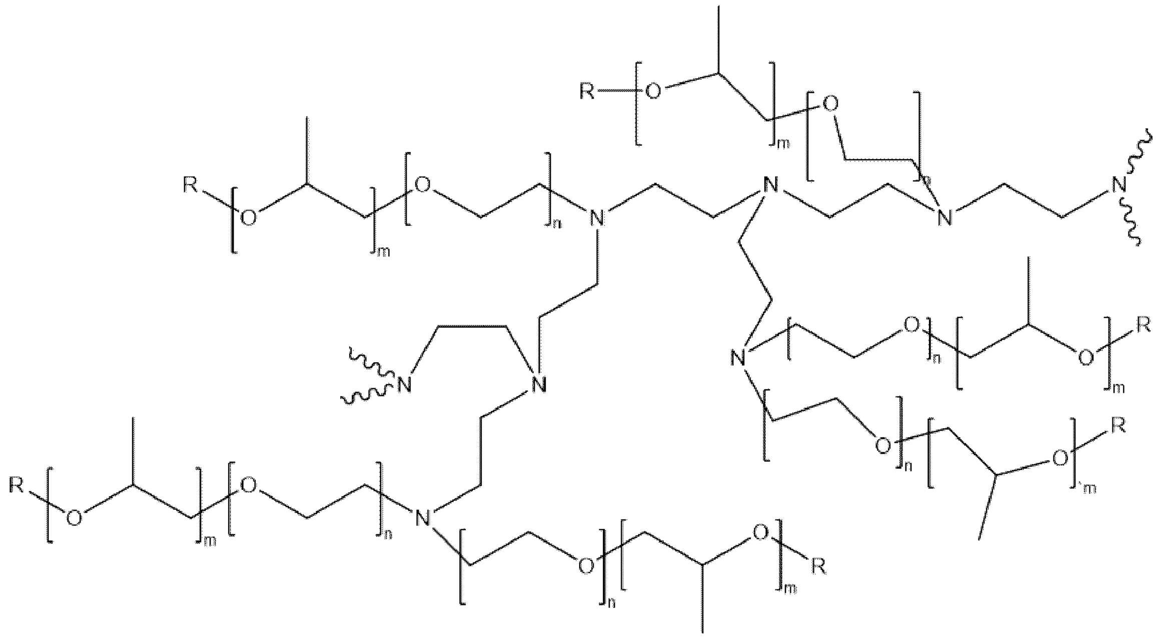

(I)

wherein the polyethyleneimine backbone has a weight average molecular weight of 600, n of formula (I) has an average of 10, m of formula (I) has an average of 7 and R of formula (I) is selected from hydrogen, a $C_1$-$C_4$ alkyl and mixtures thereof, preferably hydrogen. The degree of permanent quaternization of formula (I) may be from 0% to 22% of the polyethyleneimine backbone nitrogen atoms. The molecular weight of this amphiphilic alkoxylated polyethyleneimine polymer preferably is between 10,000 and 15,000 Da.

More preferably, the amphiphilic alkoxylated polyethyleneimine polymer has the general structure of formula (I) but wherein the polyethyleneimine backbone has a weight average molecular weight of 600 Da, n of Formula (I) has an average of 24, m of Formula (I) has an average of 16 and R of Formula (I) is selected from hydrogen, a $C_1$-$C_4$ alkyl and mixtures thereof, preferably hydrogen. The degree of permanent quaternization of Formula (I) may be from 0% to 22% of the polyethyleneimine backbone nitrogen atoms and is preferably 0%. The molecular weight of this amphiphilic alkoxylated polyethyleneimine polymer preferably is between 25,000 and 30,000, most preferably 28,000 Da.

The amphiphilic alkoxylated polyethyleneimine polymers can be made by the methods described in more detail in PCT Publication No. WO 2007/135645.

Cyclic Polyamine

The composition can comprise a cyclic polyamine having amine functionalities that helps cleaning. The composition of the invention preferably comprises from 0.1% to 3%, more preferably from 0.2% to 2%, and especially from 0.5% to 1%, by weight of the composition, of the cyclic polyamine.

The cyclic polyamine has at least two primary amine functionalities. The primary amines can be in any position in the cyclic amine but it has been found that in terms of grease cleaning, better performance is obtained when the primary amines are in positions 1,3. It has also been found that cyclic amines in which one of the substituents is -CH3 and the rest are H provided for improved grease cleaning performance.

Accordingly, the most preferred cyclic polyamine for use with the cleaning composition of the present invention are cyclic polyamine selected from the group consisting of: 2-methylcyclohexane-1,3-diamine, 4-methylcyclohexane-1,3-diamine and mixtures thereof. These specific cyclic polyamines work to improve suds and grease cleaning profile throughout the dishwashing process when formulated together with the surfactant system of the composition of the present invention.

Suitable cyclic polyamines can be supplied by BASF, under the Baxxodur tradename, with Baxxodur ECX-210 being particularly preferred.

A combination of the cyclic polyamine and magnesium sulfate is particularly preferred. As such, the composition can further comprise magnesium sulfate at a level of from 0.001 % to 2.0 %, preferably from 0.005 % to 1.0 %, more preferably from 0.01 % to 0.5 % by weight of the composition.

Triblock Copolymer

The composition of the invention can comprise a triblock copolymer. The triblock co-polymers can be present at a level of from 0.1% to 10%, preferably from 0.5% to 7.5%, more preferably from 1% to 5%, by weight of the total composition. Suitable triblock copolymers include alkylene oxide triblock co-polymers, defined as a triblock co-polymer having alkylene oxide moieties according to Formula (I): (EO)x(PO)y(EO)x, wherein EO represents ethylene oxide, and each x represents the number of EO units within the EO block. Each x can independently be on average of from 5 to 50, preferably from 10 to 40, more preferably from 10 to 30. Preferably x is the same for both EO blocks, wherein the "same" means that the x between the two EO blocks varies within a maximum 2 units, preferably within a maximum of 1 unit, more preferably both x's are the same number of units. PO represents propylene oxide, and y represents the number of PO units in the PO block. Each y can on average be from between 28 to 60, preferably from 30 to 55, more preferably from 30 to 48.

Preferably the triblock co-polymer has a ratio of y to each x of from 3:1 to 2:1. The triblock co-polymer preferably has a ratio of y to the average x of 2 EO blocks of from 3:1 to 2:1. Preferably the triblock co-polymer has an average weight percentage of total EO of between 30% and 50% by weight of the tri-block co-polymer. Preferably the triblock co-polymer has an average weight percentage of total PO of between 50% and 70% by weight of the triblock co-polymer. It is understood that the average total weight % of EO and PO for the triblock co-polymer adds up to 100%. The triblock co-polymer can have an average molecular weight of between 2060 and 7880, preferably between 2620 and 6710, more preferably between 2620 and 5430, most preferably between 2800 and 4700. Average molecular weight is determined using a 1H NMR spectroscopy (*see* Thermo scientific application note No. AN52907).

Triblock co-polymers have the basic structure ABA, wherein A and B are different homopolymeric and/or monomeric units. In this case A is ethylene oxide (EO) and B is propylene oxide (PO). Those skilled in the art will recognize the phrase "block copolymers" is synonymous with this definition of "block polymers".

Triblock co-polymers according to Formula (I) with the specific EO/PO/EO arrangement and respective homopolymeric lengths have been found to enhance suds mileage performance of the liquid hand dishwashing detergent composition in the presence of greasy soils and/or suds consistency throughout dilution in the wash process.

Suitable EO-PO-EO triblock co-polymers are commercially available from BASF such as Pluronic® PE series, and from the Dow Chemical Company such as Tergitol™ L series. Particularly preferred triblock co-polymer from BASF are sold under the tradenames Pluronic® PE6400 (MW ca 2900, ca 40wt% EO) and Pluronic® PE 9400 (MW ca 4600, 40 wt% EO). Particularly preferred triblock co-polymer from the Dow Chemical Company is sold under the tradename Tergitol™ L64 (MW ca 2700, ca 40 wt% EO).

Preferred triblock co-polymers are readily biodegradable under aerobic conditions.

The composition of the present invention may further comprise at least one active selected from the group consisting of: salt, hydrotrope, organic solvent, and mixtures thereof.

Salt:

The composition of the present invention may comprise from 0.05% to 2%, preferably from 0.1% to 1.5%, or more preferably from 0.5% to 1%, by weight of the total composition of a salt, preferably a monovalent or divalent inorganic salt, or a mixture thereof, more preferably selected from: sodium chloride, sodium sulfate, and mixtures thereof. Sodium chloride is most preferred.

Hydrotrope:

The composition of the present invention may comprise from 0.1% to 10%, or preferably from 0.5% to 10%, or more preferably from 1% to 10% by weight of the total composition of a hydrotrope or a mixture thereof, preferably sodium cumene sulfonate.

Organic Solvent:

The composition can comprise from 0.1% to 10%, or preferably from 0.5% to 10%, or more preferably from 1% to 10% by weight of the total composition of an organic solvent. Suitable organic solvents include organic solvents selected from the group consisting of: alcohols, glycols, glycol ethers, and mixtures thereof, preferably alcohols, glycols, and mixtures thereof. Ethanol is the preferred alcohol. Polyalkyleneglycols, especially polypropyleneglycol (PPG), are the preferred glycol. The polypropyleneglycol can have a molecular weight of from 400 to 3000, preferably from 600 to 1500, more preferably from 700 to 1300. The polypropyleneglycol is preferably poly-1,2-propyleneglycol.

Adjunct Ingredients

The cleaning composition may optionally comprise a number of other adjunct ingredients such as builders (preferably citrate), chelants, conditioning polymers, other cleaning polymers, surface modifying polymers, structurants, emollients, humectants, skin rejuvenating actives, enzymes, carboxylic acids, scrubbing particles, perfumes, malodor control agents, pigments, dyes, opacifiers, pearlescent particles, inorganic cations such as alkaline earth metals such as Ca/Mg-ions, antibacterial agents, preservatives, antioxidants, viscosity adjusters (e.g., salt such as NaCl, and other mono-, di- and trivalent salts) and pH adjusters and buffering means (e.g. carboxylic acids such as citric acid, HCl, NaOH, KOH, alkanolamines, carbonates such as sodium carbonates, bicarbonates, sesquicarbonates, and alike).

If present, the composition comprises from 0.01% to 2.0%, preferably from 0.05% to 1.5%, or more preferably from 0.1% to 1.0%, by weight of alkaline earth metal ions, with magnesium and/or calcium ions being particularly preferred. Low levels of transition metal ions can also be present in the liquid detergent composition, such as up to 1.0%, or up to 0.5% by weight of the composition.

Packaged product

The hand dishwashing detergent composition can be packaged in a container, typically plastic containers. Suitable containers comprise an orifice. Typically, the container comprises a cap, with the orifice typically comprised on the cap. The cap can comprise a spout, with the orifice at the exit of the spout. The spout can have a length of from 0.5 mm to 10 mm.

The orifice can have an open cross-sectional surface area at the exit of from 3 $mm^2$ to 20 $mm^2$, preferably from 3.8 $mm^2$ to 12 $mm^2$, more preferably from 5 $mm^2$ to 10 $mm^2$, wherein the container further comprises the composition according to the invention. The cross-sectional surface area is measured perpendicular to the liquid exit from the container (that is, perpendicular to the liquid flow during dispensing).

The container can typically comprise from 200 ml to 5,000 ml, preferably from 350 ml to 2000 ml, more preferably from 400 ml to 1,000 ml of the liquid hand dishwashing detergent composition.

Alternatively, the hand dishwashing detergent composition can be packaged in an inverted container. Such inverted containers typically comprise a cap at the bottom of the container, the cap comprising either a closure or a self-sealing valve, or a combination thereof. The cap preferably comprises a self-sealing valve. Suitable self-sealing valves include slit-valves. The self-sealing valve defines a dispensing orifice that opens when the pressure on the valve interior side sufficiently exceeds the pressure on the valve exterior side. The bottom dispensing container can comprise an impact resistance system, such as that described in WO2019108293A1.

Method of Washing

The resultant liquid detergent compositions can be used for manually washing. Suitable methods can comprise the steps of delivering such a liquid detergent composition to a volume of water to form a wash solution and immersing the dishware in the solution. The dishware is be cleaned with the composition in the presence of water. The dishware can be rinsed. By "rinsing", it is meant herein contacting the dishware cleaned with the process according to the present invention with substantial quantities of appropriate solvent, typically water. By "substantial quantities", it is meant usually about 1 to about 20 L, or under running water.

The composition herein can be applied in its diluted form. Soiled dishware are contacted with an effective amount, typically from about 0.5 mL to about 20 mL (per about 25 dishes being treated), preferably from about 3 mL to about 10 mL, of the cleaning composition, preferably in liquid form, of the present invention diluted in water. The actual amount of cleaning composition used will be based on the judgment of the user and will typically depend upon factors such as the particular product formulation of the cleaning composition, including the concentration of active ingredients in the cleaning composition, the number of soiled dishes to be cleaned, the degree of soiling on the dishes, and the like. Generally, from about 0.01 mL to about 150 mL, preferably from about 3 mL to about 40 mL of a cleaning composition of the invention is combined with from about 2,000 mL to about 20,000 mL, more typically from about 5,000 mL to about 15,000 mL of water in a sink. The soiled dishware is immersed in the sink containing the diluted cleaning compositions then obtained, before contacting the soiled surface of the dishware with a cloth, sponge, or similar cleaning implement. The cloth, sponge, or similar cleaning implement may be immersed in the cleaning composition and water mixture prior to being contacted with the dishware, and is typically contacted with the dishware for a period of time ranged from about 1 to about 10 seconds, although the actual time will vary with each application and user. The contacting of cloth, sponge, or similar cleaning implement to the dishware is accompanied by a concurrent scrubbing of the dishware.

Preferably, the composition is applied in its neat form to the dish to be treated. By "in its neat form", it is meant herein that said composition is applied directly onto the surface to be treated, or onto a cleaning device or implement such as a brush, a sponge, a nonwoven material, or a woven material, without undergoing any significant dilution by the user

(immediately) prior to application. Application using a sponge is preferred. "In its neat form", also includes slight dilutions, for instance, arising from the presence of water on the cleaning device, or the addition of water by the consumer to remove the remaining quantities of the composition from a bottle. Therefore, the composition in its neat form includes mixtures having the composition and water at ratios ranging from 50:50 to 100:0, preferably 70:30 to 100:0, more preferably 80:20 to 100:0, even more preferably 90:10 to 100:0 depending on the user habits and the cleaning task.

TEST METHODS

pH:

The pH is measured as a 10% aqueous solution in demineralized water at 20 °C, using a pH meter, such as an Orion Model 720A with an Ag/AgCl electrode (for example an Orion sure flow Electrode model 9172BN), calibrated using standardized pH 7 and pH 10 buffers.

Liquid detergent composition viscosity measurement (Brookfield)

The viscosity is measured at 20°C with a Brookfield RT Viscometer using spindle 31 with the RPM of the viscometer adjusted to achieve a torque of between 40% and 60%, for viscosities greater than 100 mPas. For viscosities of less than 100 mPas, spindle 18 is used.

Reserve alkalinity

The reserve alkalinity provides a measure of the capacity of the blend to neutralise any acid that is present, added, or formed *in-situ,* and hence maintain an alkaline pH. The reserve alkalinity for a solution is determined in the following manner:

The reserve alkalinity is measured on a 10% solution of the concentrated surfactant blend in deionized CO2-free water at 20°C. Deionized CO2-free water is deionized water that has been vigorously boiled for not less than 5 min, then cooled and protected from absorption of atmospheric carbon dioxide. 5g of the concentrated surfactant blend is diluted to 10% using 45g of deionized CO2-free water and mixed for at least 5 minutes until fully homogenized, to form a diluted blend. An aliquot of the diluted blend is weighed out and titrated using an automated titrator, such as the Omnis sample robot and Omnis titrator, supplied by Metrohm, using 0.1 N hydrochloric acid (HCl) and pH meter (as above), until an end-point of pH 7.0 is achieved or the maximum titration amount of the titrator is reached (25 ml for the titrator above). The volume of the titrant required to reach a pH of 7.0 is recorded, or if the maximum titration amount is reached, the final pH and volume of titrant is recorded.

The reserve alkalinity is calculated as %NaOH (g NaOH/g sample) equivalence of the number of millilitres, to the nearest 0.1 mL of 0.100 N hydrochloric acid (HCl) required for the titration to a pH of 7.0 of the sample.

The reserve alkalinity is calculated as %NaOH equivalence as follows:

$$\%NaOH = \frac{(\text{mL of } 0.100N\ HCl) \times 0.1 \times 40 \times 100}{(\text{titrated sample weight in g}) \times 1000}$$

The titrated sample weight is calculated as:

$$\text{titrated sample weight in g} = \frac{\text{weight of surfactant blend (5g)} \times \text{titrated sample weight in g}}{(\text{diluted blend weight in g}) \times 1000}$$

Where the maximum titration amount from the titrator has been reached, the final pH is recorded and the reserve alkalinity to this pH is calculated. The reserve alkalinity to pH 7.0 can then be estimated through linear extrapolation to pH 7.0 of the obtained "pH versus volume titrant" titration curves between an added titrant volume of 20ml and 25 ml.

1,4- Dioxane measurement in the concentrated surfactant blend:

The 1,4 dioxane is separated from the other components in the concentrated surfactant blend using steam distillation. The amount of 1,4-dioxane in the distillate is then measured using gas chromatography, using a flame ionization detector (GC-FID), through comparison versus a 1,3-dioxane internal reference standard.

The steam distillation is carried out using a distillation unit, such as a Foss Kjeltec™ 8100 fitted with an 800 mL

distillation reservoir. The weight of the concentrated surfactant blend sample to be subjected to steam distillation depends on the expected level of 1,4-dioxane within the concentrated surfactant blend (expressed as µg 1,4-dioxane/g 100% anionic surfactant active).

Table 1: amount of starting concentrated surfactant blend sample

| Expected 1,4-dioxane level (µg/g) | Recommended sample volume (g) |
|---|---|
| <5 | 100 |
| 5-10 | 75 |
| 10-50 | 50 |
| 50-100 | 25 |
| >100 | 10 |

The above sample volume of the concentrated surfactant blend is added to the distillation flask, together with 1ml of a 1,3-dioxane solution (0.25g 1,3-dioxane / 100ml distilled water) as the internal reference standard and 0.5 ml of a silicone antifoam (Available from Foss Product, number 67165, JTBaker B531-05).

Steam distillation is completed by the automatic addition of 100 ml distilled water at room temperature, followed by steam injection until 180 ml to 200 ml of distillate has been collected within 6 to 10 minutes of continuous addition of steam.

1 ml of the distillate is then analysed via a gas chromatography (GC-FID, using an Agilent 6890, fitted with an OVI-G43, 30 m x 0.53 mm x 3 um film Supelco column catalogue number 25396, Restek liner catalogue number 20987, and flame ionization detector), with the following settings:

| | |
|---|---|
| **Inlet** | |
| Mode | Pulsed Splitless |
| Temperature | 180°C |
| Constant flow | 5mL/min at 0.266 bar (3.86 psi), 37 cm/sec |
| Pulse Pressure | 0.689 bar (10.0 psi) |
| Pulse time | 1.00 min |
| Purge flow | 100.0mL/min |
| Purge time | 0.15 min |
| Total Flow | 107.7mL/min (approximate) |
| Gas Saver | On 15 mL/min @ 2min |
| Carrier Gas | Helium (Hydrogen or /Nitrogen can also be used with no changes to above parameters) |
| **Oven** | |
| Initial temperature | 50°C; hold for 1 minute |
| Ramp #1 | 5°C/min to 100°C; hold for 0.5 minutes |
| Ramp #2 | 25°C/min to 210°C; hold for 1.1 minutes |
| Total run time | 17 min |
| **Column** | |
| Injection volume | 0.5µl |
| **Detector** | |
| Temperature | 260°C |
| Hydrogen | 40mL/min (or as recommended by manufacturer) |
| Air | 450mL/min (or as recommended by manufacturer) |
| Makeup flow (Helium) | 45mL/min (constant column + makeup flow) (or as recommended by manufacturer) |

The 1,4-dioxane level in the concentrated surfactant blend is determined by comparing the area ratio of the 1,4-dioxane/1,3-dioxane for the test sample versus a 1,4-dioxane/1,3-dioxane calibration curve. The resulting 1,4-dioxane level was then normalized to 100% active surfactant level by taking the measured 1,4-dioxane ppm level within the concentrated surfactant blend, dividing it by the surfactant weight % active level and multiplying it by 100 to obtain the final value expressed as ppm 1,4-dioxane /100% surfactant active.

Concentrated surfactant blend viscosity:

The rheology profile is measured using a "TA instruments DHR1" rheometer, using a cone and plate geometry with a flat steel Peltier plate and a 40 mm diameter, 2.008° cone (TA instruments, serial number: SN999393). The viscosity measurement procedure includes a conditioning step and a sweep step at 20 °C. The conditioning step takes place at 20°C and consists of 10 seconds at zero shear, followed by pre-shearing for 10 seconds at 10 $s^{-1}$, followed by 30 seconds at zero shear in order for the sample to equilibrate.

An upwards shear rate sweep is conducted from a shear rate of from 0.1$s^{-1}$ to 100$s^{-1}$, in increments of 10 points per decade, each incremental step is executed by the rheometer automatically after stabilisation of the measurement. Unless otherwise stated, the viscosity is measured at shear rate of 10 $s^{-1}$ and a temperature of 20 °C.

Flow index and yield stress of the concentrated surfactant blend:

As with the viscosity measurements of the concentrated surfactant blends, the rheology profile is measured using a "TA instruments DHR1" rheometer, using a cone and plate geometry with a flat steel Peltier plate and a 40 mm diameter, 2.008° cone (TA instruments, serial number: SN999393). The viscosity measurement procedure includes a conditioning step and a sweep step at 20 °C. The conditioning step takes place at 20°C and consists of 10 seconds at zero shear, followed by pre-shearing for 10 seconds at 10 $s^{-1}$, followed by 30 seconds at zero shear in order for the sample to equilibrate.

An upwards shear rate sweep is conducted from a shear rate of from 0.1$s^{-1}$ to 100$s^{-1}$, in increments of 10 points per decade, each incremental step is executed by the rheometer automatically after stabilisation of the measurement.

The shear stress is recorded as a function of shear rate by the rheometer. The data is then fitted to the Herschley Buckley model: $\tau = \tau_0 + K \gamma^n$, where "$\tau$" is the shear stress, "$\tau_0$" is the yield stress, and is $\gamma$ the shear rate. "K" is the consistency index, and "n" is the flow index.

Concentrated surfactant blend viscosity variation with temperature

The rheology variation with temperature is measured using a "TA instruments DHR1" rheometer, using a cone and plate geometry with a flat steel Peltier plate and a 40 mm diameter, 2.026° cone (TA instruments, serial number: SN999393), at a shear rate of 10$s^{-1}$ and a temperature sweep of from 60°C down to 5°C using a temperature ramp of 2°C/min, after a 100 second equilibration step at a shear rate of 10$s^{-1}$ at 60°C.

EXAMPLES

Synthesis examples

The following blends comprising alkyl glycol sulfate and alkyl sulfate anionic surfactant were prepared as follows:

Synthesis example 1: Co-synthesis of C12 alkyl ethylene glycol sulfate and C12 alkyl sulfate blend

Co-synthesis of branched C12 alkyloxy ethanol (primarily 2-[(1-methylundecyl)oxy]ethanol (CAS 5940-87-4)) with dodecan-2-ol (CAS 10203-28-8):

To a 2-liter, single neck, round bottom reaction flask was added 50.18 g (0.298 mol) 1-dodecene, 74.08 g (1.19 mol) ethylene glycol, 49.84 g (0.262 mol) p-toluene sulfonic acid monohydrate and a magnetic stir bar. With mixing while venting to the atmosphere, the reaction flask was heated using a silicon oil bath maintained at 130°C. After 2 hours, the reaction mixture was sampled for thin layer chromatography (TLC) analysis. The TLC results indicated product formation as well as the presence of residual starting materials. The reaction flask was removed from the oil bath and allowed to cool to room temperature (21°C) at which point the reaction product was combined with 1 L aqueous solution of 10 wt% sodium carbonate, then washed three times with 2 L of ethyl acetate in a separatory funnel. The ethyl acetate layers were combined, dried by adding anhydrous sodium sulfate, filtered to remove the sodium sulfate and concentrated by evaporating solvent using rotary evaporator. The product was purified by silica gel column chromatography (80:20 hexane:ethyl acetate mobile phase), with the fractions containing the desired product being combined and concentrated

by evaporation of solvent using the rotary evaporator to yield 4.012g of product. The presence of the desired 2-[(1-methylundecyl)oxy]ethanol and dodecan-2-ol products was confirmed by NMR and MS analysis.

A second synthesis and purification was completed on similar scale using the above procedure to yield 4.672 g of desired product. The two separately synthesized/purified 2-[(1-methylundecyl)oxy]ethanol and dodecan-2-ol blends were combined for subsequent sulfation.

Co-sulfation of the branched C12 alkyloxy ethanol and dodecan-2-ol blend:

A 1-L, 3-neck, round bottom flask was equipped with a magnetic stir bar, an addition funnel with pressure equalizing arm and nitrogen gas feed in the center neck, a thermometer in one side neck and a tubing vent line in the other side neck leading to a gas bubbler filled with demineralised water to trap HC1 gas evolved from reaction. A 2-Liter glass trap was positioned between the gas bubbler and the reaction flask to prevent water being drawn back into the reaction flask. 135.5 grams of the 2-[(1-methylundecyl)oxy]ethanol and dodecan-2-ol blend, and 150 ml of ACS Reagent Grade diethyl ether (EMD Millipore product # EX0190) were added to the round bottom flask. 72.6 grams (0.617 moles) of 99.0% chlorosulfonic acid (Sigma-Aldrich product # 571024) was added to the addition funnel. A nitrogen gas flow ran from the top of additional funnel, through the flask and out the side neck vent line to the gas bubbler. An ice/NaCl/water bath was placed around the reaction flask. Mixing was begun, forming a clear, dark orange solution. Once the reaction mixture reached 5 °C, the chlorosulfonic acid was dripped in at a rate that controlled the exotherm heat release and maintained the temperature at or below 10° C. The chlorosulfonic acid addition was completed within 60 minutes.

The ice/NaCl/water bath was replaced with a 22° C water bath. The vent line tube attached to the gas bubbler was switched to a vacuum tube attached to a diaphragm vacuum pump. Solvent traps cooled with a dry ice/isopropanol bath were positioned along the vacuum tube between the reaction flask and the vacuum pump to trap volatiles pulled from the reaction mixture. A dial pressure gauge (from US Gauge reading from 0-30 inches of Hg) was positioned in the vacuum tube after the solvent trap to measure vacuum pulled on system. The reaction continued to mix for 14 minutes under nitrogen gas sweep, while the vacuum system was set up.

With continued mixing, the vacuum pump is turned on to begin applying a vacuum to the reaction mixture. The vacuum level was slowly increased by incrementally decreasing the nitrogen gas flow from the addition funnel. This was done to control foaming of the reaction mixture. Eventually the nitrogen flow was completely stopped resulting in full vacuum applied to the reaction mixture (30 inches of Hg [1.02 bar] measured on the vacuum gauge indicating full vacuum applied). Full vacuum was reached 27 minutes from start of vacuum treatment. Mixing was continued under full vacuum for an additional 26 minutes at which point the reaction mixture was clear, brown in color, fluid with minimal bubbling observed, at which point the vacuum was broken with nitrogen gas flow.

With good vortex mixing using a magnetic stir bar, the reaction mixture was slowly poured into a solution of 144.8 grams (0.676 moles) of 25.2 wt% sodium methoxide in methanol reagent (Sigma-Aldrich product # 156256) diluted to a total volume of 1 Liter with ACS Reagent Grade methanol (EMD Millipore product # MX0475) contained in a glass beaker to convert the sulfated 2-[(1-methylundecyl)oxy]ethanol and dodecan-2-ol reaction product from the acid sulfate to the sodium salt. The resulting product was a cloudy mixture with a light brown precipitate with good vortex mixing. Approximately 0.2 grams of this neutralized reaction product was dissolved in approximately 0.5 grams of demineralised water and the pH was measured to be approximately 10-11 using a pH test strip. The resulting product was mixed for an additional 15 minutes, and the pH again measured to be 10-11.

The reaction product was concentrated by evaporating the solvent using a rotary evaporator with a water bath set at 50° C until mixture began to foam, at with point the mixture was poured into a glass crystallizing dish. The crystallizing dish was placed in a vacuum oven at 21° C under partial vacuum with a slow nitrogen gas flow through the oven to prevent the product mixture from foaming while further concentrating. Internal pressure of vacuum oven was approximately 4 inches of Hg [0.14 bar]. After 4 days, the concentrated reaction product was removed from the vacuum oven. The product was now a solid. The solid was broken into smaller particles using a spatula then placed into vacuum oven at 27° C under full vacuum. After 7 hours, the product was ground into smaller particles using a mortar and pestle before being placed into a vacuum oven overnight under full vacuum at 21°C. The next day, product was again ground into smaller particles using a mortar and pestle before being placed back into the vacuum oven under full vacuum at 27°C for 7 hours and then overnight under full vacuum at 21°C. The next day, the product was sampled for NMR analysis (Proton, Carbon and DEPT) and placed in vacuum oven under full vacuum at 21°C. The next day, the product was ground using a mechanical grinder, then placed into the vacuum oven under full vacuum at 27°C for 3 hours and then at 21° for 5 days. After 5 days, the product was sampled for proton NMR and standard cationic SO3 titration analysis before being transferred to a bottle for storage. 188.2 g of light brown solid product was obtained.

NMR Analysis: 0.0424 g of the product was dissolved in a mixture of 0.6 g of deuterium oxide + 0.3 g of methanol-d4 for NMR analysis ($^{1}H$, $^{13}C$ and DEPT). By NMR, the desired target product of 2-[(1-methylundecyl)oxy]ethanol sodium sulfate and 2-dodecanol sodium sulfate was identified.

The NMR resonances for 2-dodecanol sodium sulfate were verified by the synthesis of a reference sample of 2-dodecanol sodium sulfate via the sulfation of 2-dodecanol (Sigma-Aldrich product # D221503) using the standard sulfation procedure described above.

Quantification of the level of C12 alkyl ethylene glycol sulfate and C12 alkyl sulfate in the sulfated product was determined using $^{1}H$ NMR. The level of the alkyl ethylene glycol sulfate anionic surfactant was calculated to be 86.5% by weight of the sulfated product, primarily 2-[(1-methylundecyl)oxy]ethanol sodium sulfate (i.e. where R1 is methyl and R2 is n-decyl, determined by $^{13}C$ NMR). The level of 2-dodecanol sulfate anionic surfactant was calculated to be 13.5 % by weight of the sulfated product.

The final product was determined to be 89.6% active total sulfated surfactant blend by standard cationic SO3 titration analysis (ASTM International Standard Designation: D3049), on a solids basis. The remaining 10.4% non-surfactant solids balance were impurities such as sodium sulfate, sodium chloride and residual moisture.

<u>Synthesis example 2: Co-synthesis of C14 alkyl glycol sulfate and C14 alkyl sulfate blend</u> Co-synthesis of branched C14 alkyloxy ethanol (primarily 2-[(1-methyltridecyl)oxy]ethanol (CAS# 19494-32-7)) with tetradecan-2-ol (CAS 4706-81-4):

To a 2-liter, single neck, round bottom reaction flask was added 150g (0.765 mol) 1-tetradecene, 142g (2.28 mol) ethylene glycol, 138 g (0.726 mol) p-toluene sulfonic acid monohydrate and a magnetic stir bar. With mixing while venting to the atmosphere, the reaction flask was heated using a silicone oil bath maintained at 100°C. After 72 hours, the reaction mixture was sampled for TLC analysis. TLC results indicated product formation as well as the presence of residual starting materials. The reaction flask was removed from the oil bath and allowed to cool to room temperature (21°C) at which point the reaction product was combined with 2 L aqueous solution of 10 wt% sodium carbonate, then washed three times with 1.5 L of ethyl acetate in a separatory funnel. The ethyl acetate layers were combined, dried by adding anhydrous sodium sulfate, filtered to remove sodium sulfate, then concentrated by evaporating solvent using rotary evaporator. The product was purified by silica gel column chromatography (80:20 hexane:ethyl acetate mobile phase), with the fractions containing the desired product being combined and concentrated by evaporation of solvent using rotary evaporator to yield 16 g of product. The presence of the desired products 2[(1-methyltridecyl)oxy]ethanol and tetradecan-2-ol was confirmed by NMR and MS analysis.

A second synthesis and purification was completed on similar scale using the above procedure to yield 4 g of product. The two separately synthesized/purified 2[(1-methyltridecyl)oxy]ethanol and tetradecan-2-ol blends were combined for subsequent sulfation.

Co-sulfation of the branched C14 alkyloxy ethanol and tetradecan-2-ol blend:

A 250-ml, 3-neck, round bottom flask was equipped with a magnetic stir bar, an addition funnel with pressure equalizing arm and nitrogen gas feed in the center neck, a thermometer in one side neck and a tubing vent line in the other side neck leading to a gas bubbler filled with demineralised water to trap HC1 gas evolved from reaction. A 2-Liter glass trap was positioned between the gas bubbler and the reaction flask to prevent water being drawn back into the reaction flask. 20.702 grams of the 2-[(1-methyltridecyl)oxy]ethanol and tetradecan-2-ol blend, and 85 ml of ACS Reagent Grade diethyl ether (EMD Millipore product # EX0190) were added to the round bottom flask. 9.852 grams (0.0842 moles) of 99.6% chlorosulfonic acid (Sigma-Aldrich product # 571024) was added to the addition funnel. A nitrogen gas flow ran from the top of additional funnel, through the flask and out the side neck vent line to the gas bubbler. An ice/NaCl/water bath was placed around the reaction flask. Mixing was begun, forming a clear, pale yellow solution. Once the reaction mixture reached 7 °C, the chlorosulfonic acid was dripped in at a rate that controlled the exotherm heat release and maintained the temperature at or below 10° C. The chlorosulfonic acid addition was completed within 16 minutes.

The ice/NaCl/water bath was replaced with a 22° C water bath. The vent line tube attached to the gas bubbler was switched to a vacuum tube attached to a diaphragm vacuum pump. Solvent traps cooled with a dry ice/isopropanol bath were positioned along the vacuum tube between the reaction flask and the vacuum pump to trap volatiles pulled from the reaction mixture. A dial pressure gauge (from US Gauge reading from 0-30 inches of Hg) was positioned in the vacuum tube after the solvent trap to measure vacuum pulled on system. The reaction continued to mix for 14 minutes under nitrogen gas sweep, while the vacuum system was set up.

With continued mixing, the vacuum pump is turned on to begin applying a vacuum to the reaction mixture. The vacuum level was slowly increased by incrementally decreasing the nitrogen gas flow from the addition funnel. This was done to control foaming of the reaction mixture. Eventually the nitrogen flow was completely stopped resulting in full vacuum applied to the reaction mixture (30 inches of Hg [1.02 bar] measured on the vacuum gauge indicating full vacuum applied). Full vacuum was reached 24 minutes from start of vacuum treatment. Mixing was continued under full vacuum for an additional 11 minutes at which point the reaction mixture was clear, dark orange in color, fluid with minimal bubbling observed, at which point the vacuum was broken with nitrogen gas flow.

With good vortex mixing using a magnetic stir bar, the reaction mixture was slowly poured into a solution of 19.764

grams (0.0922 moles) of 25.2 wt% sodium methoxide in methanol reagent (Sigma-Aldrich product # 156256) diluted with 85 ml of ACS Reagent Grade methanol (EMD Millipore product # MX0475) contained in a 500-ml, single neck, round bottom flask to convert the sulfated 2-[(1-methyltridecyl)oxy]ethanol and tetradecan-2-ol reaction product from the acid sulfate to the sodium salt. The resulting product was a cloudy mixture with a white precipitate with good vortex mixing. Approximately 0.2 grams of this neutralized reaction product was dissolved in approximately 0.5 grams of demineralised water and the pH was measured to be approximately 10 using a pH test strip. The resulting product was mixed for an additional 15 minutes, and the pH again measured to be 10.

The reaction product was concentrated by evaporating the solvent using a rotary evaporator with a water bath set at 50° C to obtain a soft, yellow solid. The product was placed in a vacuum oven at approximately 27° C under full vacuum for 1 hour, then allowed to go overnight under full vacuum at 21° C. The next day, the product was broken into smaller particles using a spatula then placed into vacuum oven at 27° C under full vacuum. After 7 hours, the product was ground into smaller particles using a mortar and pestle before being placed into a vacuum oven overnight under full vacuum at 21°C. The next day, product was sampled for NMR analysis then placed back into the vacuum oven under full vacuum at 21°C for 3 additional days before being transferred to a bottle for storage. 27.5 g of light brown solid product was obtained.

NMR Analysis: 0.0360 g of the product was dissolved in a mixture of 0.6 g of deuterium oxide + 0.3 g of methanol-d4 for NMR analysis ($^1$H, $^{13}$C and DEPT). By NMR, the desired target product of 2-[(1-methyltridecyl)oxy]ethanol sodium sulfate and 2-tetradecanol sodium sulfate was identified.

Quantification of the level ofC14 alkyl ethylene glycol sulfate and C14 alkyl sulfate in the sulfated product was determined using $^1$H NMR. The level of the alkyl glycol sulfate anionic surfactant was calculated to be 94.8% by weight of the sulfated product, primarily 2-[(1-methyltridecyl)oxy]ethanol sodium sulfate (i.e. where R1 is methyl and R2 is n-dodecyl, determined by $^{13}$C NMR). The level of 2-tetradecanol sulfate anionic surfactant was calculated to be 5.2 % by weight of the sulfated product.

The final product was determined to be 90.33% active total sulfated surfactant blend by standard cationic SO3 titration analysis (ASTM International Standard Designation: D3049), on a solids basis. The remaining 9.67% non-surfactant solids balance were impurities such as sodium sulfate, sodium chloride and residual moisture.

<u>Synthesis example 3: Co-synthesis of C16 alkyl glycol sulfate and C16 alkyl sulfate blend</u> Co-synthesis of branched C14 alkyloxy ethanol (primarily 2-[(1-methylpentadecyl)oxy]ethanol (CAS# 30714-96-6)) with hexadecan-2-ol (CAS 14852-31-4):

To a 2-liter, single neck, round bottom reaction flask was added 100.73 g (0.453 mol) 1-hexadecene, 112.81 g (1.82 mol) ethylene glycol, 77.80 g (0.409 mol) p-toluene sulfonic acid monohydrate and a magnetic stir bar. With mixing while venting to the atmosphere, the reaction flask was heated using a silicone oil bath maintained at 130°C. After 7 hours, the reaction mixture was sampled for TLC analysis. TLC results indicated product formation as well as the presence of residual starting materials. The reaction flask was removed from the oil bath and allowed to cool to room temperature (21°C) at which point the reaction product was combined with 2 L aqueous solution of 10 wt% sodium carbonate, then washed three times with 1.5 L of ethyl acetate in a separatory funnel. The ethyl acetate layers were combined, dried by adding anhydrous sodium sulfate, filtered to remove sodium sulfate, then concentrated by evaporating solvent using rotary evaporator. The product was purified by silica gel column chromatography (80:20 hexane:ethyl acetate mobile phase), with the fractions containing the desired product being combined and concentrated by evaporation of solvent using rotary evaporator to yield 3.91 g of product. The presence of the desired products 2-[(1-methylpentadecyl)oxy]ethanol and hexadecan-2-ol was confirmed by NMR and MS analysis.

A second synthesis and purification was completed on similar scale using the above procedure to yield 4.51 g of product. The two separately synthesized/purified 2-[(1-methylpentadecyl)oxy]ethanol and hexadecan-2-ol blends were combined for subsequent sulfation.

Co-sulfation of the branched C16 alkyloxy ethanol and hexadecan-2-ol blend:

Sulfation and neutralization were completed using the same procedure as before.

Quantification of the level ofC16 alkyl ethylene glycol sulfate and C16 alkyl sulfate in the sulfated product was determined using $^1$H NMR. The level of the alkyl ether sulfate anionic surfactant was calculated to be 83.6% by weight of the sulfated product, primarily 2-[(1-methylpentadecyl)oxy]ethanol sodium sulfate (i.e. where R1 is methyl and R2 is n-tetradecyl, determined by $^{13}$C NMR). The level of 2-hexadecanol sulfate anionic surfactant was calculated to be 16.4 % by weight of the sulfated product.

The final product was determined to be 87.5% active total sulfated surfactant blend by standard cationic SO3 titration analysis (ASTM International Standard Designation: D3049), on a solids basis. The remaining 12.5% non-surfactant solids balance were impurities such as sodium sulfate, sodium chloride and residual moisture.

Synthesis example 4: Co-synthesis of C14 alkyl glycol sulfate and C14 alkyl sulfate blend with isolation of pure C14 alkyl glycol sulfate

Co-synthesis of branched C14 alkyloxy ethanol (primarily 2-[(1-methyltridecyl)oxy]ethanol (CAS# 19494-32-7)) with tetradecan-2-ol (CAS 4706-81-4) with isolation of pure C14 alkyl glycol sulfate:

To a 500 ml, single neck, round bottom reaction flask was added 250g (1.27 mol) 1-tetradecene, 236g (3.80 mol) ethylene glycol, 208g (1.09 mol) p-toluene sulfonic acid monohydrate and a magnetic stir bar. With mixing while venting to the atmosphere, the reaction flask was heated using a silicone oil bath maintained at 120-130°C. After 48 hours, the reaction mixture was sampled for TLC analysis. TLC results indicated product formation as well as the presence of residual starting materials. The reaction flask was removed from the oil bath and allowed to cool to room temperature (21°C) at which point the reaction product was combined with 2 L aqueous solution of 10 wt% sodium carbonate, then washed three times with 1.5 L of ethyl acetate in a separatory funnel. The ethyl acetate layers were combined, dried by adding anhydrous sodium sulfate, filtered to remove sodium sulfate, then concentrated by evaporating solvent using rotary evaporator. The product was purified by silica gel column chromatography (80:20 hexane:ethyl acetate mobile phase), with the fractions containing the desired product being combined and concentrated by evaporation of solvent using rotary evaporator to yield 25.6 g of product. The presence of the desired products 2[(1-methyltridecyl)oxy]ethanol and tetradecan-2-ol were confirmed by NMR analysis.

Two additional synthesis and purification were completed on similar scale using the above procedure to yield a total of 75.57g of product. The three separately synthesized/purified 2[(1-methyltridecyl)oxy]ethanol and tetradecan-2-ol blends were combined for subsequent sulfation.

Co-sulfation of the branched C14 alkyloxy ethanol and tetradecan-2-ol blend:

A 500-ml, 3-neck, round bottom flask was equipped with a magnetic stir bar, an addition funnel with pressure equalizing arm and nitrogen gas feed in the center neck, a thermometer in one side neck and a tubing vent line in the other side neck leading to a gas bubbler filled with demineralised water to trap HC1 gas evolved from reaction. A 2-Liter glass trap was positioned between the gas bubbler and the reaction flask to prevent water being drawn back into the reaction flask. 74.656 grams of the 2-[(1-methyltridecyl)oxy]ethanol and tetradecan-2-ol blend, and 150 ml of ACS Reagent Grade diethyl ether (EMD Millipore product # EX0190) were added to the round bottom flask. 35.5 grams (0.304 moles) of 99.6% chlorosulfonic acid (Sigma-Aldrich product # 571024) was added to the addition funnel. A nitrogen gas flow ran from the top of the additional funnel, through the flask and out the side neck vent line to the gas bubbler. An ice/NaCl/water bath was placed around the reaction flask. Mixing was begun, forming a clear, dark orange solution. Once the reaction mixture reached 5 °C, the chlorosulfonic acid was dripped in at a rate that controlled the exotherm heat release and maintained the temperature at or below 10° C. The chlorosulfonic acid addition was completed within 35 minutes.

The addition funnel was removed and replaced with a nitrogen gas feed. Continued the nitrogen gas flow through the flask to the gas bubbler. The ice/NaCl/water bath was replaced with a 22° C water bath. The vent line tube attached to the gas bubbler was switched to a vacuum tube attached to a diaphragm vacuum pump. Solvent traps cooled with a dry ice/isopropanol bath were positioned along the vacuum tube between the reaction flask and the vacuum pump to trap volatiles pulled from the reaction mixture. A dial pressure gauge (from US Gauge reading from 0-30 inches of Hg) was positioned in the vacuum tube after the solvent trap to measure vacuum pulled on system. The reaction continued to mix for 20 minutes under nitrogen gas sweep, while the vacuum system was set up.

With continued mixing, the vacuum pump is turned on to begin applying a vacuum to the reaction mixture. The vacuum level was slowly increased by incrementally decreasing the nitrogen gas flow. This was done to control foaming of the reaction mixture. Eventually the nitrogen flow was completely stopped resulting in full vacuum applied to the reaction mixture (30 inches of Hg [1.02 bar] measured on the vacuum gauge indicating full vacuum applied). Full vacuum was reached 38 minutes from start of vacuum treatment. Mixing was continued under full vacuum for an additional 10 minutes at which point the reaction mixture was clear, brown in color, fluid with minimal bubbling observed, at which point the vacuum was broken with nitrogen gas flow.

With good vortex mixing using a magnetic stir bar, the reaction mixture was slowly poured into a solution of 71.2 grams (0.331 moles) of 25.2 wt% sodium methoxide in methanol reagent (Sigma-Aldrich product # 156256) diluted with 250 ml of ACS Reagent Grade methanol (EMD Millipore product # MX0475) contained in a 500-ml glass beaker to convert the sulfated 2-[(1-methyltridecyl)oxy]ethanol and tetradecan-2-ol reaction product from the acid sulfate to the sodium salt. The resulting product was a cloudy mixture with a white precipitate with good vortex mixing. Approximately 0.2 grams of this neutralized reaction product was dissolved in approximately 0.5 grams of demineralised water and the pH was measured to be approximately 11 using a pH test strip. The resulting product was mixed for an additional 15 minutes, and the pH again measured to be 11.

The reaction product was concentrated by evaporating the solvent using a rotary evaporator with a water bath set

at 50° C until mixture began to foam, at with point the mixture was poured into a glass crystallizing dish. The crystallizing dish was placed in a vacuum oven at 21° C under partial vacuum with a slow nitrogen gas flow through the oven to prevent the product mixture from foaming while further concentrating. Internal pressure of vacuum oven was approximately 2 inches of Hg [0.068 bar]. The next day, the concentrated reaction product was removed from the vacuum oven. The product was now a soft solid. The solid was broken into smaller particles using a spatula then placed into vacuum oven at 27° C under full vacuum for 6 hours, then overnight under full vacuum at 21°C. The next day, the solid product was broken into smaller particles using a spatula then placed in vacuum oven under full vacuum at 27°C for 7 hours and then overnight under full vacuum at 21°C. The next day, the product was still a soft solid at which point it was transferred to a bottle for storage. 101 g of brown, soft solid product was obtained. The product was sampled for NMR (Proton, Carbon and DEPT) and standard cationic SO3 titration analysis.

NMR Analysis: 0.0363 g of the product was dissolved in a mixture of 0.6 g of deuterium oxide + 0.3 g of methanol-d4 for NMR analysis ($^1$H, $^{13}$C and DEPT). By NMR, the desired target product of 2-[(1-methyltridecyl)oxy]ethanol sodium sulfate and 2-tetradecanol sodium sulfate was identified.

The product was determined to be 81.1% active sulfated surfactant blend by standard cationic SO3 titration analysis (ASTM International Standard Designation: D3049), on a solids basis.

Purification of the 2-[(1-methyltridecyl)oxy]ethanol sodium sulfate and 2-tetradecanol sodium sulfate blend:

To a 1-L, single neck, round bottom flask equipped with a magnetic stir bar was added 101g of the 2-[(1-methyltridecyl)oxy]ethanol sodium sulfate and 2-tetradecanol sodium sulfate blend product and 271g of Methanol. Stoppered flask and mixed for 2.5 hours at 21°C with good vortex mixing at which point the solid product was well dispersed as fine particles. Vacuum filtered mixture through Grade 4 filter paper and washed solid filter cake twice with 50 ml of 5°C methanol. The solid filter cake was transferred to a glass crystallizing dish and placed in a vacuum oven at 21°C under full vacuum. The next day, the solid product was ground into finer particles using a mortar and pestle and placed into vacuum oven at 21°C under full vacuum. After three days, the product was removed and transferred to a bottle for storage. 46.1 g of off-white solid product was obtained. The product was sampled for NMR (Proton, Carbon and DEPT) and standard cationic SO3 titration analysis.

NMR Analysis: 0.04 g of the product was dissolved in a mixture of 0.6 g of deuterium oxide + 0.3 g of methanol-d4 for NMR analysis ($^1$H, $^{13}$C and DEPT). By NMR, the desired target product of 2-[(1-methyltridecyl)oxy]ethanol sodium sulfate was identified in high purity with all major impurities removed including full removal of 2-tetradecanol sodium sulfate.

The product was determined to be 89.76% active 2-[(1-methyltridecyl)oxy]ethanol sodium sulfate by standard cationic SO3 titration analysis (ASTM International Standard Designation: D3049), on a solids basis. The remaining 10.24% non-surfactant solids balance were impurities such as sodium sulfate, sodium chloride and residual moisture.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. A process for making a concentrated surfactant blend, wherein the concentrated surfactant blend comprises a blend of at least one alkyl sulfated anionic surfactant and at least one alkyl glycol sulfate anionic surfactant, wherein the process comprises the following steps:

   a. providing an alcohol stream, wherein the alcohol stream comprises: at least one alkyl glycol and at least one alkyl alcohol, wherein:

      i. the alkyl glycol has the formula:

$$R1CH(R2)(OCH_2CH_2)_nOH \qquad (I)$$

      wherein:

         R1 is independently H, alkyl, alkylene, or a mixture thereof;
         R2 is independently alkyl, alkylene, or a mixture thereof;
         the sum of the carbon atoms present in R1 and R2 is on average from 7 to 19;

n is from 1 to 3, wherein 90 mol% or greater of the alkyl glycol of structure (I) have an n of 1 and 10 mol% or less of the alkyl glycol of structure (I) have an n of 2 or greater;

b. sulphating the alcohol stream to provide an alkyl sulfuric acid stream comprising at least one alkyl sulfuric acid and at least one alkyl glycol sulfuric acid;
c. neutralising the alkyl sulfuric acid stream to provide a concentrated anionic surfactant blend, wherein the concentrated anionic surfactant blend comprises the at least one alkyl sulfated anionic surfactant and the at least one alkyl glycol sulfate anionic surfactant, wherein the concentrated surfactant blend has a pH of at least 7.0 when measured as a 10% by weight solution of the concentrated surfactant blend in demineralized water at 20 °C.

**2.** The process according to claim 1, wherein, the alkyl glycol is derived from at least one alkene, preferably at least one linear alpha olefin (LAO), more preferably wherein the linear alpha olefin is selected from the group consisting of: 1-dodecene, 1-tetradecene, 1-hexadecene, and mixtures thereof.

**3.** The process according to claim 2, wherein, the alkyl glycol is formed via the acid-catalysed addition of ethylene glycol to the alkene.

**4.** The process according to any preceding claim, wherein in the alkyl glycol of formula (I), R1 comprises on average from 1 to 6 carbon atoms, preferably from 1 to 3 carbon atoms,
more preferably wherein in at least 80%, more preferably in at least 90% by mol of the alkyl glycol of formula (I), R1 comprises 1 carbon atom.

**5.** The process according to any preceding claim, wherein in the alkyl glycol of formula (I), R2 comprises on average from 6 to 19 carbon atoms, preferably from 8 to 17 carbon atoms, more preferably from 9 to 15 carbon atoms, most preferably from 10 to 13 carbon atoms, preferably wherein R2 is linear.

**6.** The process according to any preceding claim, wherein the alcohol in the alcohol stream consists of the alkyl glycol and the alkyl alcohol.

**7.** The process according to any preceding claim, wherein in the alcohol stream, the alkyl glycol and the alkyl alcohol can be present in a weight ratio of from 1:8 to 2:1, preferably from 1:7 to 1:1, more preferably from 1:5 to 1:2.

**8.** The process according to any preceding claim, wherein:

a. the alkyl alcohol has a number average alkyl chain length of from 8 to 18, preferably from 10 to 14, more preferably from 12 to 14, most preferably from 12 to 13 carbon atoms;
b. the alkyl alcohol has an average degree of alkoxylation of less than 1.0, preferably less than 0.5, more preferably less than 0.25, even more preferably less than 0.1, and most preferably wherein the alkyl sulfated anionic surfactant is free of alkoxylation;
c. the alkyl alcohol has an average degree of branching of less than 60%, preferably less than 40%, more preferably less than 20%, even more preferably less than 10%; most preferably is free of branching, and
d. combinations thereof.

**9.** The process according to any preceding claim, wherein the sulfation is completed to a degree of sulfation of at least 95%, preferably at least 96%, more preferably at least 98%.

**10.** The process according to any preceding claim, wherein the sulfation step is carried out in a liquid-gas interface reactor, preferably a falling film reactor.

**11.** The process according to any preceding claim, wherein during the neutralisation step, the neutralising agent is added at a level to provide the resultant concentrated surfactant blend with a pH of from 7.1 to 12, more preferably from 7.3 to 9.5, most preferably from 7.5 to 9.0, measured as a 10% by weight solution of the concentrated surfactant blend in demineralized water, at 20°C.

**12.** The process according to any of claims 1 to 11, wherein a buffering surfactant is added before or during the neutralising step, wherein:

a. the buffering surfactant is added at a level to provide the resulting concentrated surfactant blend with a reserve

alkalinity of greater than 0.02 when measured as a 10% by weight solution of the concentrated surfactant blend in demineralized water at 20°C, wherein the buffering surfactant is selected from the group consisting of: amine oxide surfactant, betaine surfactant, and mixtures thereof, preferably amine oxide surfactant, wherein the reserve alkalinity is calculated as %NaOH (g NaOH/g sample) equivalence of the number of millilitres, to the nearest 0.1 mL of 0.100 N hydrochloric acid (HCl) required for the titration to a pH of 7.0 of the sample, using the method described herein; and

b. the neutralising agent is added at a level to provide the resultant concentrated surfactant blend with a pH of from 7.1 to 10, more preferably from 7.3 to 9.5, most preferably from 7.5 to 9.0, measured as a 10% by weight solution of the concentrated surfactant blend in demineralized water, at 20°C.

**13.** A concentrated surfactant blend, wherein the concentrated surfactant blend comprises anionic surfactant at a level of from 20% to 80%, wherein the concentrated surfactant blend comprises:

a. alkyl glycol sulfate anionic surfactant, wherein the alkyl glycol sulfate anionic surfactant has the formula:

$$R1CH(R2)(OCH_2CH_2)_nOSO3^-M^+ \qquad (II)$$

wherein:

R1 is independently H, alkyl, alkylene, or a mixture thereof;
R2 is independently alkyl, alkylene, or a mixture thereof;
the sum of the carbon atoms present in R1 and R2 is on average from 7 to 19;
n is from 1 to 3, wherein 90 mol% or greater of the surfactant molecules of structure (I) have an n of 1 and 10 mol% or less of the surfactant molecules of structure (I) have an n of 2 or greater; and
M+ is a counterion; and

b. alkyl sulfated anionic surfactant, wherein the alkyl sulfated anionic surfactant has an average degree of alkoxylation of less than 3.0, preferably less than 2.0, more preferably less than 1.0, most preferably is free of alkoxylation.

**14.** The concentrated surfactant blend, wherein the blend has a viscosity of from 5,000 mPa·s to 25,000 mPa·s, preferably from 7,500 mPa·s to 20,000 mPa·s, most preferably from 10,000 mPa·s to 15,000 mPa·s, measured at a shear rate of 10 $s^{-1}$ and a temperature of 20° C.

**15.** The concentrated surfactant blend, wherein the blend has:

a. a flow index of from 0.1 to 1.0, preferably from 0.2 to 0.5, more preferably from 0.2 to 0.4; and
b. a yield stress of from 5.0 Pa to 30 Pa, preferably from 10 Pa to 25 Pa, more preferably from 15 Pa to 20 Pa.

Europäisches Patentamt
European Patent Office
Office européen des brevets

# EUROPEAN SEARCH REPORT

Application Number
EP 24 22 3146

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | WO 2024/063990 A1 (DOW GLOBAL TECHNOLOGIES LLC [US]; ROHM & HAAS [US]) 28 March 2024 (2024-03-28)<br>* paragraphs [0006] - [0008], [0013], [0018] *<br>* examples *<br>* claims *<br>----- | 1-15 | INV.<br>C11D1/37<br>C07C303/24<br><br>ADD.<br>C11D1/29<br>C11D1/14 |
| X,D | WO 2024/064645 A1 (DOW GLOBAL TECHNOLOGIES LLC [US]) 28 March 2024 (2024-03-28)<br>* page 2, line 5 - page 3, line 6 *<br>* page 8, lines 1-15 *<br>* page 18, line 23 - page 21, line 19 *<br>* page 22; table 1 *<br>* claims *<br>----- | 1-15 | |
| X | WO 2014/072840 A1 (GALAXY SURFACTANTS LTD [IN]) 15 May 2014 (2014-05-15)<br>* page 5, lines 1-7 *<br>* page 6, lines 3-21 *<br>* examples 1, 3-7, 10, 11 *<br>* claims *<br>----- | 1-15 | TECHNICAL FIELDS SEARCHED (IPC)<br><br>C11D<br>C07C |

The present search report has been drawn up for all claims

1

| Place of search | Date of completion of the search | Examiner |
|---|---|---|
| The Hague | 25 June 2025 | Bertran Nadal, Josep |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................................................................
& : member of the same patent family, corresponding document

EPO FORM 1503 03.82 (P04C01)

## ANNEX TO THE EUROPEAN SEARCH REPORT ON EUROPEAN PATENT APPLICATION NO.

EP 24 22 3146

This annex lists the patent family members relating to the patent documents cited in the above-mentioned European search report. The members are as contained in the European Patent Office EDP file on
The European Patent Office is in no way liable for these particulars which are merely given for the purpose of information.

25-06-2025

| Patent document cited in search report | Publication date | Patent family member(s) | Publication date |
|---|---|---|---|
| WO 2024063990 A1 | 28-03-2024 | AR 130373 A1 | 04-12-2024 |
| | | CN 120019135 A | 16-05-2025 |
| | | EP 4577630 A1 | 02-07-2025 |
| | | WO 2024063990 A1 | 28-03-2024 |
| WO 2024064645 A1 | 28-03-2024 | AU 2023347853 A1 | 01-05-2025 |
| | | CN 119866327 A | 22-04-2025 |
| | | EP 4577522 A1 | 02-07-2025 |
| | | WO 2024064645 A1 | 28-03-2024 |
| WO 2014072840 A1 | 15-05-2014 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2024063990 A1
- WO 202463991 A1
- WO 202464645 A
- WO 202464645 A1
- JP 2006104438 A
- JP 2006137872 A
- EP 0850907 A1
- WO 2011002284 A
- WO 2007135645 A
- WO 2019108293 A1


### Non-patent literature cited in the description


- **BAKKER P M**. Sulfonates and sulfates of sec-alkyl ethyl ether: detergents prepared by the addition of substituted alcohols to 1-alkenes. *CHIMIE, PHYSIQUE ET APPLICATIONS PRATIQUES DES AGENTS DE SURFACT, XX, XX*, 09 September 1968, 157-165
- **J. F. KNIFTON**. Detergent-range alcohol alkoxylates via vicinal glycol additions to alpha-olefins. *APPLIED CATALYSIS, A*, 1995, vol. 130, 79-88
- **P. M. BAKKER**. An exploratory study of the addition reactions of ethyleneglycol, 2-chloroethanol and 1,3-dichloro-2-propanol to 1-dodecene. *JOURNAL OF THE AMERICAN OIL CHEMISTS' SOCIETY*, September 1967, vol. 44, 517-521
- **YU, H.** ; **WANG, C** ; **LIN, T et al.** Direct production of olefins from syngas with ultrahigh carbon efficiency. *Nat. Commun.*, 2022, vol. 13, 5987
- **KLAUS NOWECK**. *Wolfgang Grafahrend*, 2006
- Fatty Alcohols. Ullmann's Encyclopedia of Industrial Chemistry. Wiley-VCH
- Ziegler Alcohol Synthesis (Ziegler Higher Alcohol Synthesis, Alfol Process, Ziegler-Alfol Process, Ziegler-Alfol Synthesis). **ZERONG WANG**. Comprehensive Organic Name Reactions and Reagents. John Wiley & Sons, Inc, 2010
- **WEBER, HERMAN** ; **; FALBE, JURGEN**. Oxo Synthesis Technology. *Industrial & Engineering Chemistry*, April 1970, vol. 62 (4), 33-37
- **WAGNER, JOHN D** ; **LAPPIN, GEORGE R** ; **ZIETZ, J. RICHARD**. Alcohols, Higher Aliphatic, Synthetic Processes. *Kirk-Othmer Encyclopedia of Chemical Technology*, 2000
- **KREUTZER, U.R**. Manufacture of fatty alcohols based on natural fats and oils. *J Am Oil Chem Soc*, 1984, vol. 61, 343-348
- **W. HERMAN DE GROOT**. Sulf(on)ation Technology in the Detergent Industry. Springer-Science+Business Media, 1991
- *CHEMICAL ABSTRACTS*, 5940-87-4
- *CHEMICAL ABSTRACTS*, 10203-28-8